# EUROPEAN PATENT SPECIFICATION

(11) **EP 2 547 252 B1**
(45) Date of publication and mention of the grant of the patent: **02.06.2021**
(21) Application number: 11756645.5
(22) Date of filing: 17.03.2011
(51) Int. Cl.: A61B 5/0402, A61B 5/0408, A61B 5/0452

(54) **ELECTROCARDIOGRAPHIC MONITORING SYSTEM**
ELEKTROKARDIOGRAPHISCHES ÜBERWACHUNGSSYSTEM
SYSTÈME DE SURVEILLANCE ÉLECTROCARDIOGRAPHIQUE

(30) Priority: 17.03.2010 US 314628 P
(43) Date of publication of application: 23.01.2013
(73) Proprietor: Web Biotechnology Pte Ltd, Singapore 408564 (SG)
(72) Inventor: SHIM, Se Ngie Winston, Singapore 128805 (SG); WONG, En Hou Philip, Singapore 099200 (SG); LOO, Yong Ying, Singapore 753357 (SG); PUAH, Soon Hiang, Singapore 760114 (SG)
(74) Representative: Viering, Jentschura & Partner mbB Patent- und Rechtsanwälte
(86) International application number: PCT/SG2011/000105
(87) International publication number: WO 2011/115579

(56) References cited:
- EP-A2- 0 865 762
- EP-A2- 1 495 716
- WO-A1-02/089667
- WO-A1-2007/085068
- WO-A2-2007/092543
- WO-A2-2009/112972
- US-A- 3 878 833
- US-A- 4 137 908
- US-A- 4 253 721
- US-A- 4 331 153
- US-A- 4 793 361
- US-A- 5 355 883
- US-A1- 2004 049 120
- US-A1- 2005 049 515
- US-A1- 2006 155 183
- US-A1- 2006 235 476
- US-A1- 2006 282 007
- US-A1- 2007 149 887
- US-A1- 2008 139 953
- US-A1- 2009 264 792
- US-B1- 7 149 576

## Description

### Field Of The Invention

The invention relates to an electrocardiographic (ECG or EKG) monitoring system.

### Background Of The Invention

Heart disease is one of the most common morbidities. The cost of care for recovering patients in hospital and social/economic burden from patients with underlying heart disease that is undetected has significant implication in the long-term cost of healthcare.

In 2006, the cost for healthcare in the United States rose to $2.1 trillion or 16 percent of the gross domestic product (GDP). This correlates with the fact of an aging population and an increase of chronic diseases, which account for more than 75% of the total US healthcare costs. This effect is to a large extent due to the aging society and a more sedentary lifestyle.

However, this development is not restricted to the US but is a worldwide problem that both developed and developing countries are facing. Hence, a major challenge in healthcare is to more efficiently provide high quality care for an increasing number of patients using limited financial and human resources.

On account of widely publicized sudden death cases occurring in the young and apparently healthy people during routine exercise, the need for a personalized ECG monitoring device that can be readily worn for cardiac surveillance is required.

### Summary Of The Invention

According to the invention, there is provided an electrocardiographic monitoring system as defined in independent claim 1.

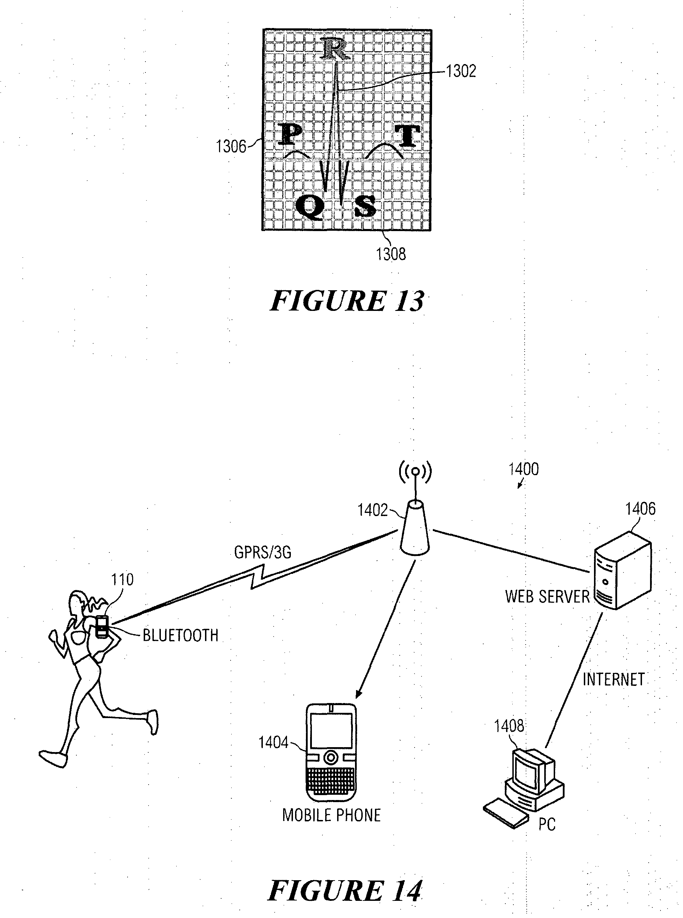

In the context of various embodiments, the term "electrocardiographic monitoring system" may refer to one or more devices used to detect and process electrocardiogram (ECG or EKG) signals, which are an electrical representation of the contractile activity of the heart over time. ECG indicates the overall rhythm of the heart and weaknesses in different parts of the heart muscle, and can measure and diagnose abnormal rhythms of the heart. An ECG signal can be represented by a cyclic occurrence of patterns with different frequency content (QRS complexes, P and T waves). These letters (Q, R, S, P and T) are arbitrary names give to five deflections in an ECG trace (see numeral 1302 in Figure 13). The Q, R and S wave occur in rapid succession and reflect a single event, so are thus normally considered as a whole complex. A P wave occurs just before the QRS complex. A Q wave is any downward deflection after a P-wave. An R-wave is an upward deflection and the S wave is any downward deflection after the R-wave. A T wave occurs just after the QRS complex.

In the context of various embodiments, the term "housing" may mean a portable structure having suitable dimensions that allow the housing to be attached to a creature body part. The housing may have a length in the range of 5 to 10cm, for example 7cm; a breadth in the range of 4 to 8cm, for example 5cm; and a height in the range of 2 to 5cm, for example around 3cm. In one embodiment, the housing may not have a uniform cross-section, whereby at greatest points, the housing has a length of 6cm, a breadth of 5.5cm and a height of 1.5cm. In the context of various embodiments, the interior of the housing contains the signal processor, the transmitter and any other electrical components required to process and transmit ECG signals, so as to monitor health status.

In the context of various embodiments, the term "electrodes" may mean any electrical conducting material having any shape (for example, strips, triangle, circle, square) and having any dimension, as long as they are able to detect ECG signals. In the context of various embodiments, the electrodes detect ECG signals in a non-invasive manner - either by direct contact on the surface of a body part; or indirect contact through docking to metallic contacts, the metallic contacts being adapted to directly contact the surface of a body part.

In the context of various embodiments, the term "boundary" refers to the perimeter of an external surface of the housing, so that the perimeter of the external surface surrounds the plurality of electrodes, although at least a portion of the plurality of electrodes may lie along the perimeter of the external surface.

In the context of various embodiments, the term "signal processor" may mean a module capable of controlling the electrical components, such as the plurality of electrodes and the transmitter, that are coupled to the module. For instance, the signal processor may receive signals from any one or more of the plurality of electrodes or transmit signals from any one or more of the plurality of electrodes. The signal processor may execute instructions to perform a logic sequence, wherein the instructions may be embedded or programmable. The logic sequence may refer to the implementation of flowcharts of instructions, the flowcharts looping at one or more portions, with the signal processor activating one or more components of the electrocardiographic monitoring system, such as the transmitter and any one or more of the plurality of electrodes. The signal processor may be an application specific integrated circuitry (ASIC) entirely fabricated on a single chip, in the context of semiconductor technology. The ASIC may have one or more devices that provide processing functions such as AND, NAND, or OR logic using transistors, resistors, capacitors, inductors and the like. The signal processor may comprise a memory which is for example used in the processing carried out by the signal processor. A memory used in the embodiments may be a volatile memory, for example a DRAM (Dynamic Random Access Memory) or a non-volatile memory, for example a PROM (Programmable Read Only Memory), an EPROM (Erasable PROM), EEPROM (Electrically Erasable PROM), or a flash memory, e.g., a floating gate memory, a charge trapping memory, an MRAM (Magnetoresistive Random Access Memory) or a PCRAM (Phase Change Random Access Memory).

In the context of various embodiments, the term "signal" may refer to ECG signals or any other signals that may be used in the monitoring of the ECG signals.

According to one aspect of the disclosure, there is provided a method of locating a QRS complex within ECG data, the method including: receiving a signal having ECG data; detecting a first occurring peak value; detecting a first minimum value occurring prior to the first occurring peak value; comparing the first occurring peak value and the minimum value against pre-determined QRS complex parameters; and denoting the first occurring peak value and the minimum value as R and Q locations respectively when both the first occurring peak value and the minimum value match the R and Q parameters of the pre-determined QRS complex parameters.

In the context of various embodiments, the term "pre-determined QRS complex parameters" may refer to pre-set cut-offs with QRS duration greater than 70ms and QRS amplitude greater than 250uV.

According to one aspect of the disclosure, there is provided a docking interlace between an electrical contact and an electrode lead, the docking interface including an electrical contact provided on a housing having dimensions allowing attachment to a creature body part; an electrode lead adapted to contact a creature body part; and a fastening mechanism provided on either or both of the electrical contact and the electrode lead to allow fastening between the electrical contact and the electrode lead.

In the context of various embodiments, the term "docking interface" may refer to a structure for allowing an ECG signal detecting device to be removably attached to a body part where an ECG signal is to be detected. In the context of various embodiments, the term "electrical contact" may refer to electrodes, located on an ECG signal detecting device that is adapted to be removably attached to a body part where an ECG signal is to be detected, the electrodes allowing an ECG signal to be transmitted for processing by electrical components within the ECG signal detecting device. In the context of various embodiments, the term "electrode lead" may refer to electrodes that are in direct contact with a body part where an ECG signal is to be detected; the electrodes allowing the electrical contact to fasten thereto, so that the electrode lead and the electrical contact are separate structures. In the context of various embodiments, the term "fastening mechanism" may refer to a structure that allows the electrical contact and the electrode lead to secure, in a detachable manner, onto each other.

According to one aspect of the disclosure, there is provided circuitry to reduce noise within an ECG signal, the circuitry including a primary input configured to receive a first ECG signal; a reference input configured to receive a second ECG signal; and an adaptive filter having a transfer function, the adaptive filter coupled to the primary input and the reference input, wherein the transfer function self-adjusts according to the first ECG signal and the second ECG signal to produce co-efficients for the adaptive filter that reduce noise within the first ECG signal and the second ECG signal, to output an ECG signal having reduced noise.

In the context of various embodiments, the term "circuit" may be understood as any kind of a logic implementing entity, which may be special purpose circuitry or a processor executing software stored in a memory, firmware, or any combination thereof. Thus, in an embodiment, a circuit may be a hard-wired logic circuit or a programmable logic circuit such as a programmable processor, e.g. a microprocessor (e.g. a Complex Instruction Set Computer (CISC) processor or a Reduced Instruction Set Computer (RISC) processor). A circuit may also be a processor executing software, e.g. any kind of computer program, e.g. a computer program using a virtual machine code such as e.g. Java. Any other kind of implementation of the respective functions which will be described in more detail below may also be understood as a circuit in accordance with an alternative embodiment.

### Brief Description Of The Drawings

In the drawings, like reference characters generally refer to the same parts throughout the different views. The drawings are not necessarily to scale, emphasis instead generally being placed upon illustrating the principles of the invention. In the following description, various embodiments of the invention are described with reference to the following drawings, in which:
Figure 1A is a schematic representation of an electrocardiographic monitoring system according to an embodiment.
Figure 1B is a schematic representation of an electrocardiographic monitoring system according to an embodiment.
Figure 1C shows a flow chart for a method, according to an embodiment, to locate a QRS complex within ECG data.
Figure ID is a schematic representation of an electrocardiographic monitoring system according to an embodiment.
Figure IE shows an implementation of the electrocardiographic monitoring system of Figure 1B, according to an embodiment.
Figure 2A and 2B respectively show a top view and a side view of an ECG monitoring device, according to an embodiment.
Figures 3A and 3B respectively show a bottom view and a perspective view of the ECG monitoring device, according to an embodiment.
Figure 3C shows the location of V1, V2, V3, V4, V5 and V6 ECG lead locations.
Figures 4A, 4B and 4C respectively show a top view, a bottom and a side view of a docking pad having docking electrode leads, according to an embodiment.
Figure 4D shows a schematic representation of a docking interface, according to an embodiment.
Figures 5A and 5B respectively show a top view and a bottom view of a docking pad having docking electrode leads, according to an embodiment.
Figure 6 is a schematic representation of a signal processor, according to an embodiment.
Figure 7A shows noise typically present for a conventional 12 electrode wired system that is used to measure an ECG signal.
Figure 7B shows an ECG signal having noise arising from muscular tremors.
Figure 8 is a block diagram of a Finite Impulse Response (FIR) filter, according to an embodiment.
Figure 9A is a schematic representation of circuitry, according to an embodiment, to reduce noise within an ECG signal.
Figure 9B is a block diagram of a circuitry, according to an embodiment, that can be used to address interference levels and also noise within an ECG signal.
Figure 10 shows a signal where baseline wandering is present.
Figure 11 shows a flow chart for a method, according to an embodiment, to locate a QRS complex within ECG data.
Figure 12 shows a flow chart for an algorithm implementing the method of Figure 11, according to an embodiment.
Figure 13 shows an exemplary PQRST complex.
Figure 14 shows a network where an electrocardiographic monitoring system, according to an embodiment, is implemented.
Figure 15 shows a light and mobile telecommunications tool, according to an embodiment.

### Detailed Description

Embodiments provide for a simple non-obstructive, mobile, light-weight, cable-free, electrocardiogram (ECG or EKG) monitoring device that communicates wirelessly and continuously with, for example, a mobile phone for data collection and analysis. The ECG monitoring device provides a personalized cardiac rhythm detection and heart rate monitoring device for rehabilitating patients recovering from heart disease management as well as individuals that are conscious of their health in weight management and cardiac fitness during exertional activities. The collected data may be stored in a central server for long-term data analysis and case review for disease progression that may or may not be symptomatic to the patients or person at risk for early prognosis and pre-emptive interventions.

In one possible application, the ECG monitoring device detects electrical potential from the left chest of users, digitizes and filters data including amplitude and duration of various ECG waveforms. Through long-term recording of the heart beats, irregular heart rhythm and periodic irregular heart rate will be detected at rest as well as during exertional activities. By comparing collected data against historical data of the same individual, an accurate prognosis can be made by physicians and evidence-based symptom management can be recommended for timely interventions in a tailored manner.

A system, incorporating the ECG monitoring device, can be used for continuous monitoring of heart rhythm and heart rate irregularity that may or may not be coincided with symptomic complaints (e.g. palpitation, syncope, breathlessness) of patients. Through the analysis of long-term data trends collected, a prognosis can be made on the heart condition of the patients. This may entail intensive management of ongoing disease through pharmacological means or pre-emptive interventions of developing disease (that may or may not be symptomatic to the patients).

The system allows for non-intrusive and simple monitoring. The ECG monitoring device is light-weight, mobile and cable-free, with sensors that attach to the chest of individual for direct electrical signal detection. The ECG monitoring device has, in an embodiment, three electrodes to provide for a Lead-I or Lead-II configuration. A Lead-I configuration measures the voltage between the left arm and the right arm. A Lead-II configuration measures the voltage between the left leg and the right arm. The detected signal is transmitted wirelessly, for example, via Bluetooth to a mobile phone that acts as a receiver for continuous data for data storage/data display and relaying station to a central server for in-depth data analysis and tracking of data trends.

Various embodiments also address various issues of (1) inability of ECG electrodes to pick up signal accurately as the spacing between the electrode spacing reduces significantly, (2) motion artifacts with the minimum number of electrodes and least spaced electrode arrangement, (3) presence of noise, transmission interference and heat generation in small-size packages having electronic circuits, and (4) the need for complex algorithm to baseline wandering for accurate detection of ECG waveform. Various embodiments allow for dry, wet and bio-insulated electrodes to ensure performance integrity after prolonged exposure to sweat.

It will be appreciated that common numerals, used in the relevant drawings, refer to components that serve a similar or the same purpose.

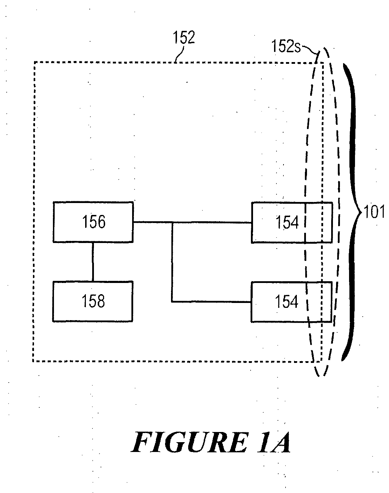

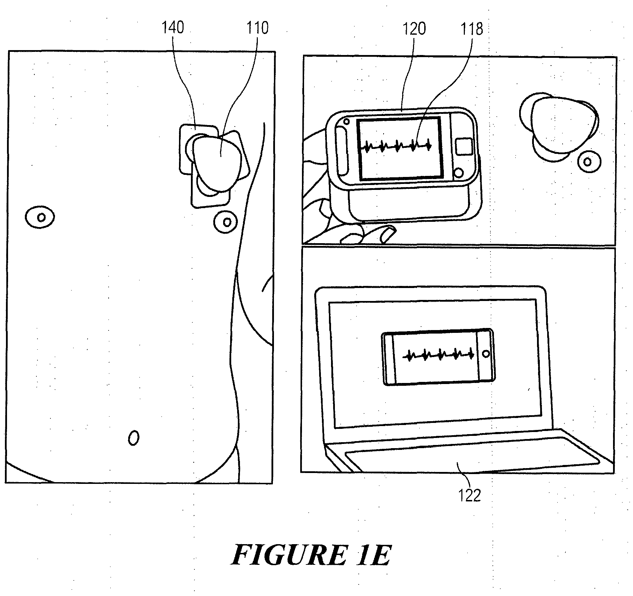

Figure 1A is a schematic representation of an electrocardiographic monitoring system 101 according to an embodiment.

In an embodiment, an electrocardiographic monitoring system 101 includes: a housing 152 configured to be attached to a creature body part. The housing 152 includes: a plurality of electrodes 154 confined within the boundary of the housing 152. The plurality of electrodes 154 are arranged a distance apart from each other and accessible from a same exterior surface 152s of the housing 152. A signal processor 156 is configured to receive signals from any one or more of the plurality of electrodes 154 and transmit signals to any one or more of the plurality of electrodes 154. A transmitter 158 is configured to transmit signals from the signal processor 156.

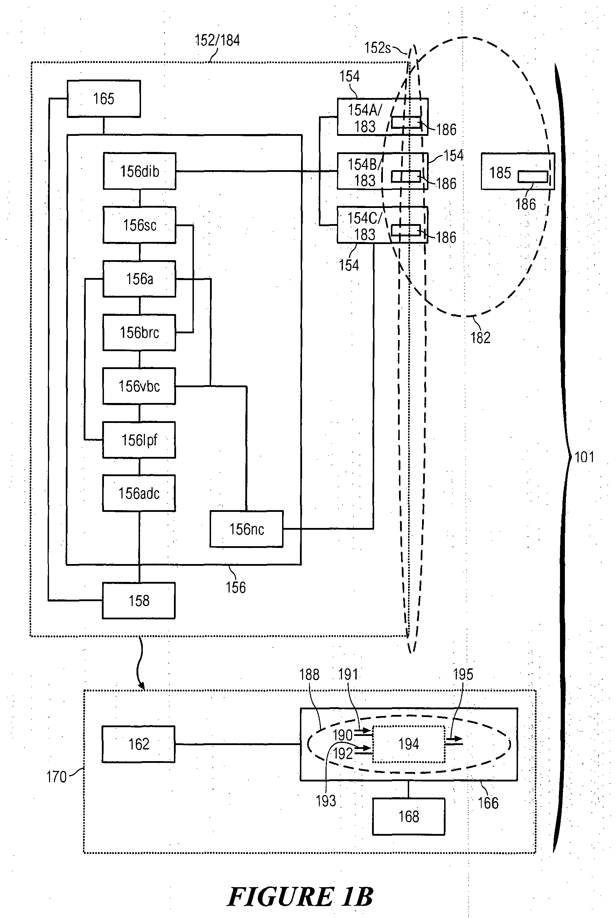

Figure 1B is a schematic representation of the electrocardiographic monitoring system 101 of Figure 1A.

Similar to Figure 1A, the electrocardiographic monitoring system 101 includes: the housing 152 configured to be attached to a creature body part. The housing 152 includes: a plurality of electrodes 154 confined within the boundary of the housing 152. The plurality of electrodes 154 are arranged a distance apart from each other and accessible from a same exterior surface 152s of the housing 152. The signal processor 156 is configured to receive signals from any one or more of the plurality of electrodes 154 and transmit signals to any one or more of the plurality of electrodes 154. The transmitter 158 is configured to transmit signals from the signal processor 156.

Figure 1B shows that the plurality of electrodes 154 may include at least three input electrodes (154A, 154B and 154C) configured to send signals to the signal processor 156. The plurality of electrodes 154 may include an electrode 154C configured as a ground terminal.

Figure 1B shows that the signal processor 156 may include a differential input buffer 156dib coupled to at least two input electrodes of the plurality of electrodes 154. The signal processor 156 may include a subtractor circuit 156sc coupled to the differential input buffer 156dib. The signal processor 156 may include an amplifier 156a coupled to the subtractor circuit 156sc. The signal processor 156 may include a baseline restoration circuit 156brc coupled to the amplifier 156a and the subtractor circuit 156sc. The signal processor 156 may include a voltage bias circuit 156vbc coupled to the amplifier 156a and the baseline restoration circuit 156brc. The signal processor 156 may include a low pass filter 1561pf coupled to the amplifier 156a and the voltage bias circuit 156vbc. The signal processor 156 may include an analogue to digital converter 156adc coupled to the low pass filter 1561pf and the transmitter 158. The signal processor 156 may include a neutral circuit 156nc coupled to the voltage bias circuit 156vbc, the neutral circuit 156nc coupled to a ground terminal electrode 154C of the plurality of electrodes 154.

The distance between each centre of the plurality of electrodes 154 may be less than 5cm. The shape of the housing may have a longest length of less than 7cm. An equal distance may exist between each centre of the plurality of electrodes 154.

The plurality of electrodes 154 may be arranged so that each electrode (154A, 154B and 154C) is located at a vertice of a triangle. The triangle may be equilateral or may be isosceles. The electrodes 154 may be arranged along a straight line.

The electrocardiographic monitoring system 101 may further include a receiver 162 to receive signals from the transmitter 158. A processor 166 is coupled to the receiver 162, the processor 166 configured to process the signals received from the transmitter 158. A display 168 is coupled to the processor 166, the display 168 configured to show ECG data from the signals received by the receiver 162.

The receiver 162, the processor 166 and the display 168 may be located in a platform 170 remote from the housing 152. The platform 170 may be any one or more of the following devices: a computer server, a mobile device and a computer terminal. The mobile device may be any one or more of the following devices: a mobile phone, a PDA, a tablet and a laptop.

In an embodiment shown in Figure 1B, the electrocardiographic monitoring system 101 may include a power source 165 to power the signal processor 156 and the transmitter 158.

The transmitter 158 transmits signals using any one or more of the following mediums: EDGE (Enhanced Data rates for GSM (Global System for Mobile Communications) Evolution); bluetooth; 3G (3rd generation); HSDPA (High-Speed Downlink Packet Access); LTE (Long Term Evolution); WiFi (wireless local area network using IEEE 802.11 communication standards); WiMax (Worldwide Interoperability for Microwave Access); protocols based on the IEEE 802.15.4-2003 standard for Low-Rate Wireless Personal Area Networks (LR-WPAN), such as Zigbee; low power RF (radio frequency) operating in ISM (industrial, scientific and medical) band or cable.

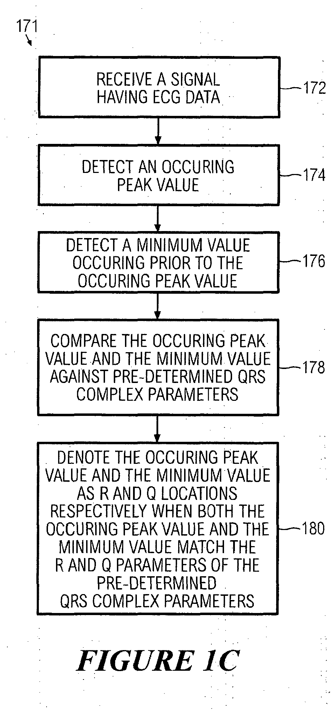

Figure 1C shows a flow chart 171 for a method, according to an embodiment, to locate a QRS complex within ECG data.

At 172, a signal having ECG data is received.

At 174, a first occurring peak value is detected.

At 176, a first minimum value occurring prior to the first occurring peak value is detected. At 178, the first occurring peak value and the minimum value are compared against pre-determined QRS complex parameters.

At 180, the first occurring peak value and the minimum value are denoted as R and Q locations respectively when both the first occurring peak value and the minimum value match the R and Q parameters of the pre-determined QRS complex parameters.

At 180, the minimum value may be denoted as the Q location only if the minimum value occurs within a pre-defined interval from the peak value.

At 174 and 176, any one or more of the first occurring peak and the first minimum value may be detected by processing the signal having the ECG data using a differential equation. The differential equation may be a first order differentiation followed by second order differentiation.

The method may further include detecting a second peak value occurring prior to the Q location and denoting the second peak value as a P location. A second minimum value occurring subsequent to the R location may be detected and the second minimum value denoted as an S location. A third peak value occurring subsequent to the S location may be detected and the third peak value denoted as a T location.

The second peak value, the second minimum value and the third peak value may be respectively denoted as the P, S and T locations only if the second peak value, the second minimum value and the third peak value occur within a pre-defined interval from the R or Q location.

Any one or more of the second peak value, the second minimum value and the third peak value may be detected by processing the signal having the ECG data using a differential equation. The differential equation may be a first order differentiation followed by second order differentiation. In an embodiment, a first derivative is obtained for the ECG signal, where the first derivative is set to zero and the resulting equation is solved to locate turning points within the ECG signal. A second derivative is then calculated to determine whether the turning point is a peak value or a minimum value.

The following process may be reiterated: detecting a peak value occurring subsequent to an earlier peak value; detecting a minimum value occurring prior to the peak value; comparing the peak value and the minimum value against pre-determined QRS complex parameters; and denoting the peak value and the minimum value as subsequent R and Q locations respectively when both the peak value and the minimum value match the R and Q parameters of the pre-determined QRS complex parameters.

The located QRS complex may be grouped into pre-determined intervals. The pre-determined intervals may be compared against the pre-determined QRS complex parameters to determine portions of the pre-determined intervals that match, the match providing an indication of a medical condition. The pre-determined interval may be a multiple of 10 seconds, for example an integer multiple of 10 seconds, such as 20 seconds and 30 seconds.

Comparing the first occurring peak value and the minimum value against pre-determined QRS complex parameters may be conducted using one or more of the following: an image processing technique, heuristic determination or using an artificial neural learning network.

The first occurring peak value and the minimum value may be matched against any one or more of the pre-determined QRS complex parameters comprising: minimum QRS amplitude, QRS interval, PR interval, PR range, QT interval, constancy of PR interval and constancy of RR interval.

Returning to Figure 1B, the electrocardiographic monitoring system 101 may further include a docking interface 182 between an electrical contact and an electrode lead.

The docking interface 182 includes an electrical contact 183 provided on a housing 184 when having dimensions allowing attachment to a creature body part, an electrode lead 185 adapted to contact a creature body part; and a fastening mechanism 186 provided on either or both of the electrical contact 183 and the electrode lead 185 to allow fastening between the electrical contact 183 and the electrode lead 185. In Figure 1B, the electrical contact 183 and the housing 184 are shown to be provided by the plurality of electrodes 154 and the housing 152 respectively. However, it will be appreciated that in another embodiment (not shown), the electrical contact 183 and the housing 184 are those from another ECG monitoring system.

The fastening mechanism 186 may include any one or more of the following structures: snap-on buttons, clips, magnets, studs, adhesive pads and hook and loop (Velcro) fasteners. The snap-on button may include a stud and popper.

Circuitry 188, to reduce noise within an ECG signal, may be provided. The circuitry 188 includes a primary input 190 configured to receive a first ECG signal 191; a reference input 192 configured to receive a second ECG signal 193; and an adaptive filter 194 having a transfer function. The adaptive filter 194 is coupled to the primary input 190 and the reference input 192, wherein the transfer function self-adjusts according to the first ECG signal 191 and the second ECG signal 193 to produce co-efficients for the adaptive filter 194 that reduce noise within the first ECG signal 191 and the second ECG signal 193, to output an ECG signal 195 having reduced noise. In the embodiment shown in Figure 1B, the circuitry 188 is provided in the processor 166. However, it is possible to have the circuitry 188 provided in the signal processor 156.

Figure ID is a schematic representation of an electrocardiographic monitoring system 100 according to an embodiment. The electrocardiographic monitoring system 100 includes an ECG monitoring device 110 that communicates and transmits wirelessly and continuously to a platform 120.

The platform 120 may be any portable computing device configured for data collection, storage and analysis, such as a mobile phone, a laptop or a tablet PC (such as the "Apple iPad"). The platform 120 may be any one or more of the following devices: a computer server, a mobile device and a computer terminal. Information in the platform 120 can then be transmitted via existing telephony networks to another remote processing device 122 (such as mobile devices or personal computers, mobile or static computer servers) for further analysis.

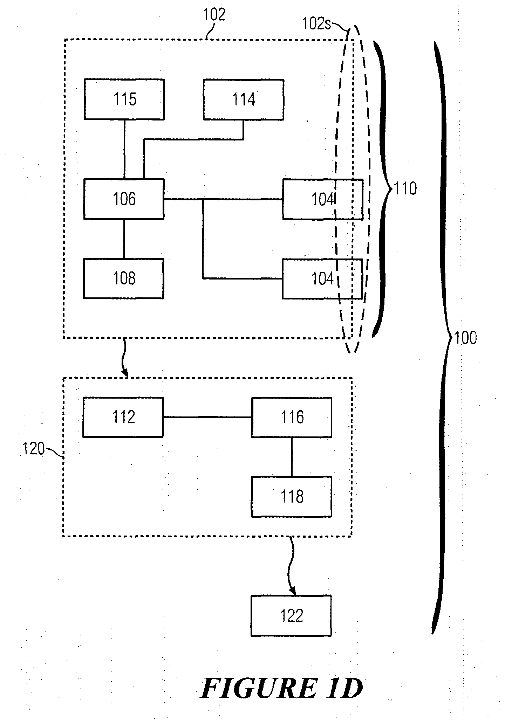

The ECG monitoring device 110 has a housing 102 configured to be attached to a creature body part. The housing 102 includes a plurality of electrodes 104 confined within the boundary of the housing 102, the plurality of electrodes arranged a distance apart from each other and accessible from a same exterior surface 102s of the housing 102. In the embodiment shown in Figure 1D, the housing 102 provides the ECG monitoring device 110 with a simple and portable design, which is non-obstructive, mobile, light-weight and cable-free.

In the embodiment shown in Figure ID, the interior of the housing contains a signal processor 106, a transmitter 108, a power source 115 and any other electrical components required to process and transmit ECG signals, so as to monitor health status. The power source 115 powers the signal processor 106 and the transmitter 108.

The signal processor 106 is configured to receive signals from any one or more of the plurality of electrodes 104 and transmit signals to any one or more of the plurality of electrodes 104. In the embodiment shown in Figure ID, the signal processor 106 processes signals received from or sent to any one or more of the plurality of electrodes 104, in accordance to a logic sequence, which may be embedded or programmable.

The transmitter 108 is configured to transmit signals from the signal processor 106. The transmission may be by any one or more of the following mediums: EDGE (Enhanced Data rates for GSM (Global System for Mobile Communications) Evolution), bluetooth, 3G (3rd generation), HSDPA (High-Speed Downlink Packet Access), LTE (Long Term Evolution), WiFi (wireless local area network using IEEE 802.11 communication standards), WiMax (Worldwide Interoperability for Microwave Access), protocols based on the IEEE 802.15.4-2003 standard for Low-Rate Wireless Personal Area Networks (LR-WPAN), such as Zigbee, low power RF (radio frequency) operating in ISM (industrial, scientific and medical) band or cable.

A receiver 112 receives signals from the transmitter 108. A processor 116 is coupled to the receiver 112, the processor 116 configured to process the signals received from the transmitter 108. A display 118 is coupled to the processor 116, the display configured 118 to show ECG data from the signals received by the receiver 112. In the embodiment shown in Figure ID, the receiver 112, the processor 116 and the display 118 are located in a platform 120 remote from the housing 102. The platform 120 may also send the ECG data to another remote processing device 122 (such as a central server) wirelessly or via cable connection.

In the embodiment shown in Figure ID, the ECG monitoring device 110 provides a personalized cardiac rhythm detection and heart rate monitoring device for rehabilitating patients recovering from heart disease management as well as for any individuals that are conscious of their health and can be used to accurately manage weight reduction and/or cardiac fitness during exertional activities. The collected data can be stored in a memory module 114 in the ECG monitoring device 110 itself (by sending a suitable signal from the signal processor 106 to the memory module 114), or the data can be stored in the platform 120 for long-term data analysis and case review for disease progression that may or may not be symptomatic to the patients or person at risk.

The ECG monitoring device 110 detects, through one or more of the plurality of electrodes 104, electrical potential from a body part which is then digitized and filtered by the signal processor 106. The data comprises amplitude and duration of various ECG waveforms. Through continuous recording of heart beat rate which the above mentioned device is capable of, irregular heart rhythm and periodic irregular heart rate can be detected at rest as well as during various exertional activities. By comparing the continuously recorded data with the historical data of the same individual, an accurate prognosis can be made by physicians and evidence-based symptom management can be recommended for timely interventions.

Figure IE shows an implementation of the electrocardiographic monitoring system 100 of Figure ID, according to an embodiment, for cable-free/wire-free electrocardiogram detection and wireless data transmission to a receiver or a computer.

In Figure IE, the ECG monitoring device 110 is attached to a creature body part 140 (Figure IE shows that the body part 140 is a portion above the left nipple of the chest of a human being). Preferably, the ECG monitoring device 110 is attached on the left side of the chest of a user to detect electrical potential of the heart between contraction cycles, wherein the electrical potential values are digitized and filtered to provide data being amplitude and duration of various ECG waveforms.

The ECG monitoring device 110 transmits wirelessly and continuously to the platform 120, being in the embodiment shown in Figure IE, a mobile handphone. The display 118 shows the ECG signal waveform that is detected from the creature body part 140. Data related to the ECG signal waveform in the platform 120 is then transmitted to the processing device 122, being in the embodiment shown in Figure IE, a laptop.

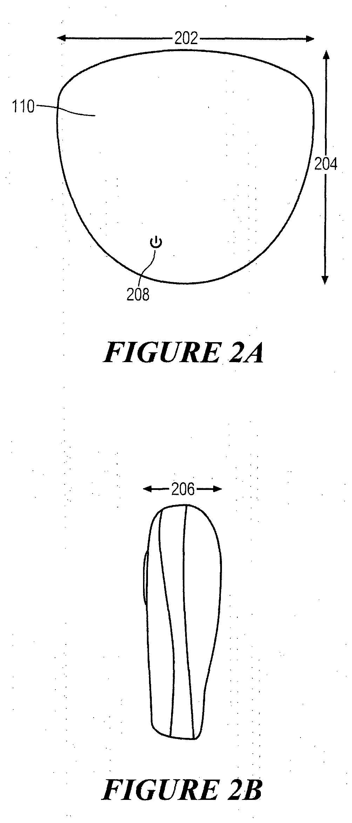

Figures 2A and 2B respectively show a top view and a side view of an ECG monitoring device, according to one embodiment. In Figures 2A and 2B, the use of the reference numeral 110 for the ECG monitoring device, being the same as the ECG monitoring device 110 of Figure 1D, is arbitrarily chosen from the ECG monitoring systems that are schematically represented in Figures 1A, 1B and ID. It will thus be appreciated that the ECG monitoring device shown in Figures 2A and 2B is in accordance with the embodiments of the ECG monitoring systems described Figures 1A, 1B and 1D.

The ECG monitoring device 110 allows for continuous monitoring, for example in the range of 5 to 24 hours, for example for around 8 hours, using a fully charged rechargeable battery, to detect heart rhythm and heart rate irregularity that may or may not be coincided with symptomic complaints (e.g. palpitation, syncope, breathlessness) from users. The monitoring period may be extended to enable continuous monitoring by charging the device while in operation or by using an external battery pack (not shown).

The housing 102 of the ECG monitoring device 110 has a length 202 in the range of 5 to 10cm, for example 7cm; a breadth 204 in the range of 4 to 8cm, for example 5cm; and a height 206 in the range of 2 to 5cm, for example around 3cm. The ECG monitoring device 110 occupies an area in the range of 2000 to 8000 mm², for example 3500mm²; and weighs in the range of 8 to 35g, for example 26g. In one embodiment, the housing may not have a uniform cross-section, whereby at greatest points, the housing has a length of 6cm, a breadth of 5.5cm and a height of 1.5cm. In this embodiment, the area of the ECG monitoring device is around 3300 mm². Other embodiments may use different dimensions 202, 204 and 206. The housing 102 may be an ergonomic casing that is water-proof and splash-proof for multi-purpose applications, including use for activities such as swimming and aerobic exercise, where sweat may result in poor insulation and interference of the ECG signal. A switch 208, provided on the top surface, allows the switching on and off of the ECG monitoring device 110.

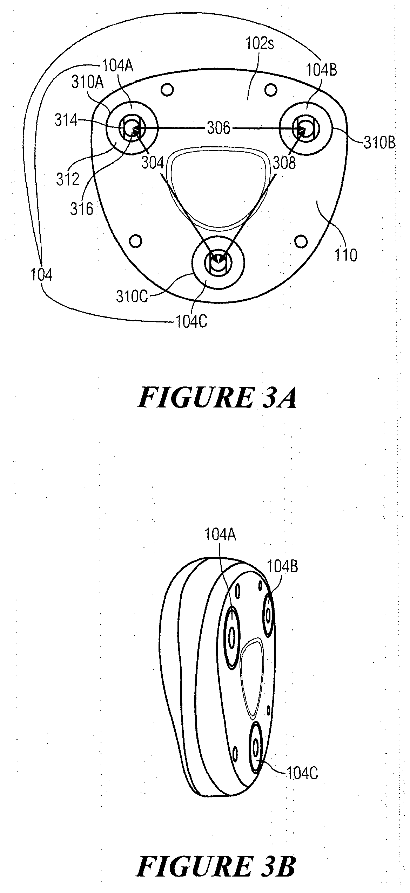

Figure 3A shows a bottom view of the ECG monitoring device 110 of Figure 2A, whereupon the plurality of electrodes 104 is located; while Figure 3B shows a perspective view of the ECG monitoring device 110 of Figure 2A, where the bottom of the ECG monitoring device 110 can be seen.

In the embodiment shown in Figure 3A, the plurality of electrodes 104 is realised by three input electrodes (104A, 104B and 104C), disposed in a 3-Lead ECG arrangement, and configured to send signals to the signal processor. While not shown, it is also possible that more than three electrodes may be used. Any one of the plurality of electrodes 104 may be configured as a ground terminal.

The plurality of electrodes 104 are confined within the boundary 302 of the housing 102 and arranged a distance apart (304, 306 and 308) from each other. Although other dimensions are possible, the distance between each centre of the plurality of electrodes 104 is about 5cm or less than 5cm.

In the embodiment shown in Figure 3A, the plurality of electrodes 104 are arranged so that each electrode (104A, 104B and 104C) is located at a vertice of a triangle.

Between two or more of the plurality of electrodes 104, an equal distance may exist between each centre of the plurality of electrodes 104. Thus, when the distances (304, 306 and 308) are equal, each of the electrodes 104A, 104B and 104C is located at a corresponding vertice of an equilateral triangle. On the other hand, when only two of the distances (304, 306 and 308) are equal, each of the electrodes 104A, 104B and 104C is located at a corresponding vertice of an isosceles triangle. In another embodiment (not shown), the plurality of electrodes are arranged along a straight line. Between any two adjacent electrodes, an equal distance may also exist between the centre of one electrode and the centre of the other electrode. It is also possible that the distance between any two adjacent electrodes is not the same.

The plurality of electrodes 104 is accessible from a same exterior surface 102s of the housing 102.

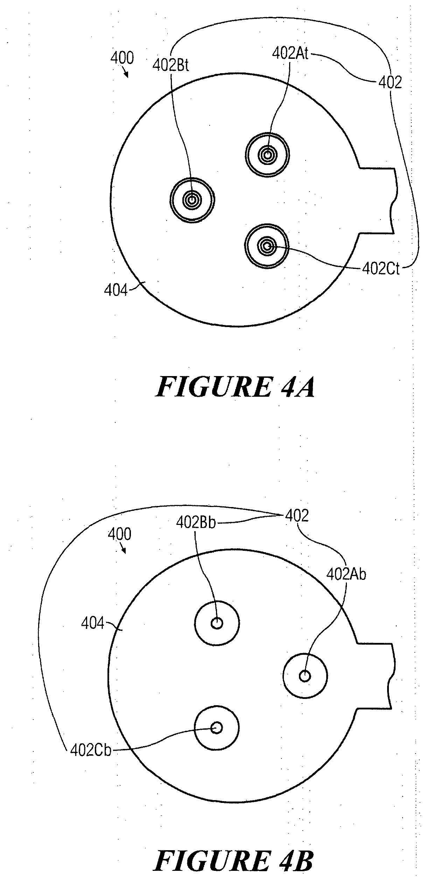

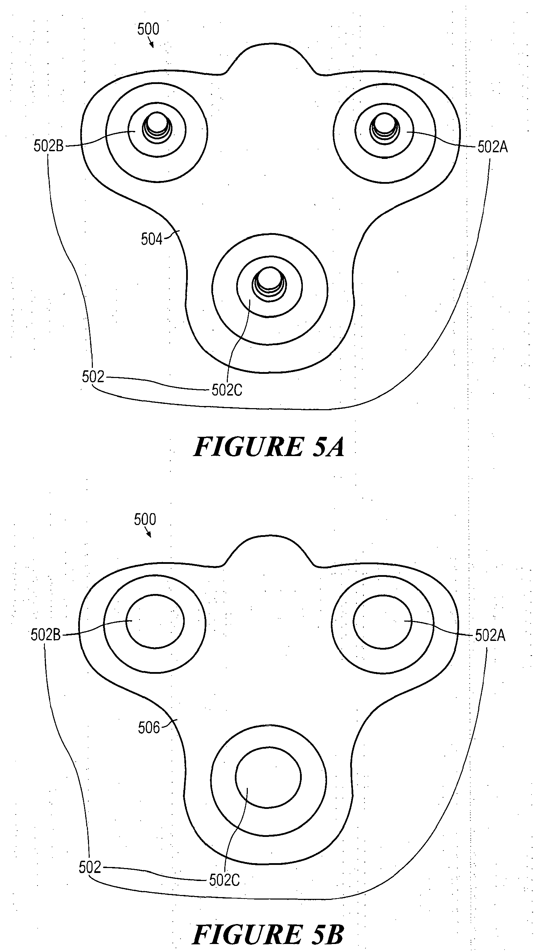

In the embodiment shown in Figure 3A, each electrode (104A, 104B and 104C) is provided in a corresponding opening (310A, 310B and 310C) on the surface 102s, so that the contact surface of each electrode (104A, 104B and 104C) is recessed relative to (or located a distance beneath, see perspective view provided in Figure 3B) the surface 102s level. The contact surface of each electrode (104A, 104B and 104C) is recessed so as to more readily accommodate and secure onto a corresponding electrode lead from a docking pad (see Figures 4A to 4C and 5A to 5B).

In another embodiment (not shown), each of the plurality of electrodes 104 may be provided directly on the exterior surface of the housing, so that the contact surface of the plurality of electrodes protrude a distance from the exterior surface level.

The plurality of electrodes may have a fastening mechanism to allow detachable securing with electrodes that are separately provided. The detachable securing may be frictional engagement, so that the separately provided electrodes are corresponding shaped to the electrodes provided on the housing of the ECG monitoring device. In the embodiment shown in Figure 3A, each of the plurality of electrodes 104 has the fastening mechanism integrated therein. Each electrode (104A, 104B and 104C) is shaped as a female component of a fastener arrangement, wherein each electrode (104A, 104B and 104C) is shaped similarly to a popper of a stud and popper arrangement. The fastener arrangement may include or may be a snap-on button stud fastener arrangement. Taking electrode 104A as an example, there is a disc-shaped part 312 surrounding a bore 316 that is adapted to accommodate a male component (not shown) of the fastener arrangement, for example of the snap-on button stud fastener arrangement. Two spring wires 314 extends across the bore 316, where each spring wire 314 traces a chord (i.e. a line segment whose endpoints lie on the bore 316) which is off-centre to the bore 316. Both of the spring wires 314 are adapted to grip the male component (not shown) of the snap-on button stud fastener. In the embodiment shown in Figure 3A, the other electrodes (104B and 104C) will have the same structural arrangement as the electrode 104A. In this manner, the plurality of electrodes 104 is adapted to secure, by a snap-on action, onto electrode leads (not shown) having a corresponding shape to the plurality of electrodes 104. Such electrode leads may have a male component of a snap-on button stud fastener. The electrode leads may be adapted to contact a body part where ECG signals are to be measured, so that when the plurality of electrodes 104 is coupled to the electrode leads, the plurality of electrodes 104 is indirectly attached to the body part. However, the plurality of electrodes 104 can also measure ECG signals through direct attachment to the body part using a suitable fastening device (not shown), such as an adhesive pad, located on the exterior surface 102s of the housing 102.

In another embodiment not part of the present invention, the plurality of electrodes do not have an integrated fastening mechanism and may simply be electrical conducting material having any shape (for example, strips, triangle, circle, square).

In use, all the electrodes (104A, 104B and 104C) of the ECG monitoring device 110 may directly contact the skin on the left side of the chest of a user. In one placement, the electrodes 104A and 104B are in parallel positions to the left collar bone and above the left nipple for a Lead-I configuration (i.e. to measure the voltage between the left arm and the right arm) of ECG signal. In another placement, the electrodes 104A and 104B may be positioned perpendicular to the left collar bone for a Lead-II configuration (i.e. to measure the voltage between the left leg and the right arm) of ECG signal. In a further placement, the ECG monitoring device 110 can be positioned for a Lead-III configuration (to measure the voltage between the right leg and the left arm) by rotating the ECG monitoring device 110 90 degrees clockwise from the Lead-II configuration. Thus, in the Lead-I to Lead-III configurations, placement of the ECG monitoring device 110 does not require extra cable.

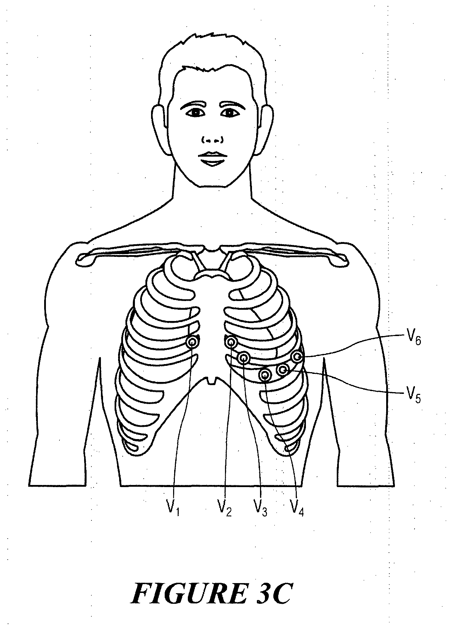

With reference to Figure 3C, V1 is located in the 4^{th} intercostal space to the right of the sternal boarder. V2 is located to the left of the sternal boarder in the 4^{th} intercostal space. V4 is located in the 5^{th} intercostal space at the left mid-clavicular line. V3 is located directly between V2 and V4. V5 is located level with V4 at the anterior axillary line. V6 is located level with V5 at the mid-axillary line.

For a V1 configuration, the ECG monitoring device 110 is placed in an orientation that is opposite of the Lead-I configuration, i.e. the ECG monitoring device 110 is rotated 180 degrees relative to how the ECG monitoring device 110 is positioned for the Lead-I configuration and placed on the right side of chest (rather than the left side). For the V2 to V6 configurations, while maintaining the orientation of the V1 configuration, a positive electrode of the three electrodes (104A, 104B and 104C) is placed at the corresponding V2 to V6 locations.

Thus, the arrangement of the electrodes (104A, 104B and 104C) allows the ECG monitoring device 110 to be placed in the Lead-I, Lead-II, Lead-III and V1 to V6 configurations without the use of additional cable.

In another placement, extra cable may be used to connect all the electrodes (104A, 104B and 104C) of the device for all other conventional configurations of ECG lead positions (e.g. aVF, aVL, aVR). For aVF (lead augmented vector foot), aVR (lead augmented vector right), aVL (lead augmented vector left) configurations, a Y-shaped wire/cable may be used to split a negative electrode of the three electrodes (104A, 104B and 104C) into two negative electrodes.

In the aVF (lead augmented vector foot) configuration, a positive electrode of the three electrodes (104A, 104B and 104C) is connected to the left leg or the middle of the body and the two negative electrodes (from the Y-shaped wire/cable) is connected to the right arm and the left arm

In the aVL (lead augmented vector left) configuration, a positive electrode of the three electrodes (104A, 104B and 104C) is connected to the left arm or the left side of the body and the two negative electrodes (from the Y-shaped wire/cable) is connected to the right arm and the right leg.

In the aVR (lead augmented vector right) configuration, a positive electrode of the three electrodes (104A, 104B and 104C) is connected to the right arm or the right side of the body and the two negative electrodes (from the Y-shaped wire/cable)is connected to the left arm and the left leg.

It is also possible to place the ECG monitoring device 110 on other places of the body to capture the required ECG signals. For example, it is possible to directly contact the electrode 104A, the electrode 104B and the electrode 104C with the left index finger, the right index finger and the middle finger of the user respectively.

In the above configurations, the three electrodes (104A, 104B and 104C) may, in one embodiment, be wired such that electrode 104A is the positive electrode, electrode 104B is the negative electrode, while electrode 104C is the neutral electrode.

Before placement of the ECG monitoring device 110, specialized electrode leads, upon which the electrodes (104A, 104B and 104C) connect to, may be placed on the user. While single electrode leads may be used with the ECG monitoring device 110, a dock having three docking electrode leads in a 3-in-1 integrated format may be used.

Figures 4A and 4B respectively show a top view and a bottom view of a docking pad 400 having a plurality of electrode leads 402 that can be used as such docking electrode leads, according to an embodiment. The docking pad 400 provides for a 3-in-1 integrated docking electrode leads for an ECG monitoring device (such as the ECG monitoring device 110 of Figures 2A to Figure 3B).

The plurality of electrode leads 402 is provided on a pad 404. The pad 404 is made from non-electrical conducting material, so as to electrically isolate each of the electrode leads 402. Each electrode lead 402 extends through the thickness of the pad 404, so that a top surface (402At, 402Bt and 402Ct; see Figure 4A) of the electrode leads 402 is provided on a top surface of the pad 404 (see Figure 4A), while a bottom surface (402Ab, 402Bb and 402Cb; see Figure 4B) is provided on a bottom surface of the pad 404 (see Figure 4B).

The bottom surfaces (402Ab, 402Bb and 402Cb; see Figure 4B) of the electrode leads 402 are adapted to be in direct contact, by being placed onto, a body part where an ECG signal is to be detected.

The top surfaces (402At, 402Bt and 402Ct; see Figure 4A) of the electrode leads 402 are adapted, by having an integrated fastening mechanism, to allow fastening thereto of an electrical contact (such as the electrodes 104A, 104B and 104C of the ECG monitoring device 110, see Figures 3A and 3B) on an ECG signal detecting device.

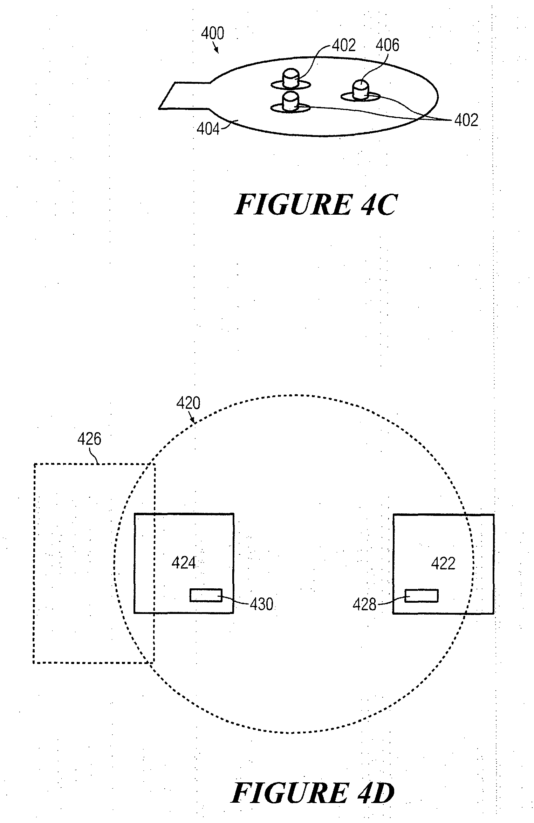

Together, between the electrical contact (such as, but not limited to, the electrodes (104A, 104B and 104C) of the ECG monitoring device 110 (see Figures 3A and 3B) and the electrode leads 402, a docking interface 420, as shown in the schematic representation of Figure 4D, is provided, allowing an ECG signal detecting device to be removably attached to a body part where an ECG signal is to be detected.

The docking interface 420, between an electrical contact and an electrode lead, includes an electrical contact 424 provided on a housing 426 (of an ECG monitoring device, such as the ECG monitoring device of Figures 3A and 3B) having dimensions allowing attachment to a creature body part. An electrode lead 422, adapted to contact a creature body part, has a fastening mechanism 428 to allow fastening between the electrical contact 424 (for example the electrodes 104, see Figure 3A) and the electrode lead 422. The fastening mechanism (see reference numeral 430 may also be provided on the electrical contact 424 to allow fastening between the electrical contact 424 and the electrode lead 422. It is also possible that the fastening mechanism (428, 430) is provided on either of the electrical contact 424 and the electrode lead 422 to allow fastening between the electrical contact 424 and the electrode lead 422.

The fastening mechanism (428, 430) is a structure that allows the electrical contact 424 and the electrode lead 422 to secure, in a detachable manner, onto each other. The fastening mechanism (428, 430) may be any one or more of the following structures: snap-on buttons, clips, magnets, studs, adhesive pads and hook and loop (Velcro) fasteners; as long as a path for an electric signal is established when the electrical contact 424 and the electrode lead 422 secure onto each other.

With reference to Figure 4C (showing a side view of the docking pad 400 having the plurality of electrode leads 402) the fastening mechanism is integrated into the electrode lead 402 and is of a snap-on button configuration, more specifically a stud and popper arrangement. A portion of each of the electrode leads 402 is shaped as a stud 406, where the stud 406 has reduced diameter compared to the portion of the electrode lead 402 that is provided on the pad 404 and the stud 406 projects from the pad 404.

The stud 406 acts as a male component of the snap-on button configuration. As an example and with reference to Figure 3A, the stud 406 is adapted to be inserted into the bore 316 of the electrode 104A, so that an enlarged portion of the stud 406 is gripped by the spring wires 314.

By having the pad 404 with the electrode leads 402, ease of use is provided, since an ECG signal can be consistently tapped from a same location after the pad 404 is attached to a body part. In addition, when used with the ECG monitoring device 110 shown in Figures 2A to 3B, the electrode leads 402 provide the electrodes (104A, 104B and 104C) of the ECG monitoring device 110 with a docking location. In one embodiment where the docking pad 400 is used in conjunction with the ECG monitoring device 110 (see Figures 3A and 3B), the specific arrangement of the electrode leads 402 and the spacing between each of the electrode leads 402 are both manufactured to correspond to where the three electrodes (104A, 104B and 104C) are located, so that the ECG monitoring device 110 can only be connected to the docking pad 400 in one orientation. In this manner, it is ensured that the ECG monitoring device 110 (see Figures 3A and 3B) is located at a same desired ECG lead position when the ECG monitoring device 110 is removed and attached from the docking pad 400. The docking pad 400 is repositioned should there be a need to have the ECG monitoring device 110 connected at another ECG lead position.

The electrode leads 402 ensure good physical contact to the skin (e.g. chest surface) and facilitate outputing clean baseline signals and reduces baseline wandering phenomenon. Motion artifacts associated with minute movement between electrode leads, skin surface and device are minimised.

In the embodiment shown in Figure 4B, the plurality of electrode leads 402 are in a dry format with insulated and isolated individual electrodes. In another embodiment, gel type or foam type electrode leads may be used to detect ECG signals.

Figures 5A and 5B respectively show a top view and a bottom view of a docking pad 500 having a plurality of electrode leads 502 that can be used as docking electrode leads, according to an embodiment.

The electrode leads 502 are similar to the electrode leads 402 described above, so no further elaboration is provided. The only difference is the shape of the pad 504 where the electrode leads 502 are mounted upon is different from that of the pad 404 where the electrode leads 402 are mounted upon. In addition; with reference to Figure 5B, the bottom surfaces of the electrode leads 502 is covered with a protective sheet 506, which is peeled off before the docking pad 500 is used.

In long-term recording of a user's heart beats, irregular heart rhythm and periodic irregular heart rate are detected at rest, as well as during exertional activities. By comparing to historical data of the same user, an accurate prognosis can be made by physicians and evidence-based symptom management can be recommended for timely interventions in a tailored manner.

For long-term data collection, clean baseline ECG signals and deterministic ECG waveform of the individual P, Q, R, S, T wave recognition are crucial. This entails, in an embodiment, a hardware configuration that performs:
i) filtering device;
ii) noise suppression;
iii) baseline wandering reduction; and
iv) QRS complex reproduction.

In the following, filtering according to various embodiments will be described.

In an embodiment, data collected by electrodes of the ECG monitoring device 110 (see Figure ID) is processed by a high resolution filtering circuit including, but not limited to, high and low pass filtering techniques to achieve a clean and near noiseless signal. In the embodiment shown in Figure 1D, such a filtering circuit may be provided in the signal processor 106.

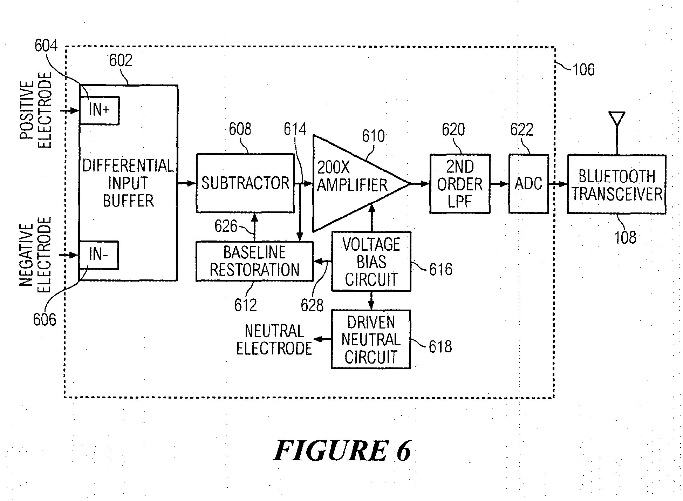

Figure 6 is a schematic representation of the signal processor 106, used in the ECG monitoring device 110 of Figure ID, according to the present invention. In Figure 6, the signal processor 106 is coupled to the transmitter 108. Raw data, present in the electrical signal captured by the device, may be sampled at a frequency rate not lower than 100Hz, to ensure good signal to noise ratio.

The signal processor 106 includes a differential input buffer 602, having two ports 604 and 606 coupled to at least two input electrodes of the plurality of electrodes 104 (see Figure 1D). For instance, with reference to Figure 3A, a first port 604 may be coupled to the electrode 104A and a second port 606 may be coupled to the electrode 104B. A subtractor circuit 608 is coupled to the differential input buffer 602. An amplifier 610 is coupled to the subtractor circuit 608. A baseline restoration circuit 612 is coupled to the amplifier 610 and the subtractor circuit 608. In the embodiment shown in Figure 6, the port 604 is a positive input, while the port 606 is a negative input, so that the electrode 104A becomes a positive electrode, while the electrode 104B becomes a negative electrode.

The differential input buffer 602, the subtractor circuit 608, the amplifier 610, the baseline restoration circuit 612 and the voltage bias circuit 616 function as follows.

At the differential input buffer 602, two high-impedance amplifiers monitor the voltage between the input and the interface ground, where the first port 604 provides an input for one of the two high-impedance amplifiers, while the second port 606 provides an input for the other of the two high-impedance amplifiers. The outputs of the two amplifiers are then subtracted by a third amplifier, at the subtractor circuit 608, to remove any signal common to both the input signal at the first port 604 and the input signal at the second port 606. The signal difference between the input signal at the first port 604 and the input signal at the second port 606 forms a baseline signal 614, which is sent to both the amplifier 610 and the baseline restoration circuit 612. In the baseline restoration circuit 612, the baseline signal 614 and a biasing voltage signal 628 from the voltage bias circuit 616 is compared and a resulting correction signal 626 fed back to the subtractor circuit 608. Since the biasing voltage signal 628 from the voltage bias circuit 616 is stable, the biasing voltage signal 628 is used to correct baseline swings in the baseline signal 614. Thus, the subtractor circuit 608, the voltage bias circuit 616 and the baseline restoration circuit 612 serve to maintain the baseline.

The voltage bias circuit 616 is coupled to the amplifier 610 and the baseline restoration circuit 612. The voltage bias circuit 616 provides a bias voltage to the right leg and also serves to provide a baseline voltage to the amplifier 610 and the baseline restoration circuit 612. A low pass filter 620 is coupled to the amplifier 610. Furthermore, the low pass filter 620 may be coupled to the voltage bias circuit 616 through another module (not shown). An analogue to digital converter 622 is coupled to the low pass filter 620 and the transmitter 108.

The amplifier 610 amplifies the baseline signal 614. Subsequently, the low pass filter 620 removes signals higher than a pre-determined frequency from the amplified baseline signal. In one embodiment, the pre-determined frequency may be for example in the range of 50Hz to 60 Hz. The analogue to digital converter 622 then digitizes the analogue output from the low pass filter 620.

A neutral circuit 618 is coupled to the voltage bias circuit 616, where the neutral circuit 618 is coupled to a ground terminal electrode of the plurality of electrodes 104 (see Figure ID), such as with reference to Figure 3A, the electrode 104C, so that the electrode 104C becomes a neutral electrode.

While not shown, it is possible that the signal processor 106 includes a logic circuit to which the ports 604 and 606 and the neutral circuit 618 is connected, the logic circuit being able to automatically designate which of the three electrodes (104A, 104B and 104C) will be a positive, negative or a neutral electrode.

In the following, noise suppression according to various embodiments will be described.

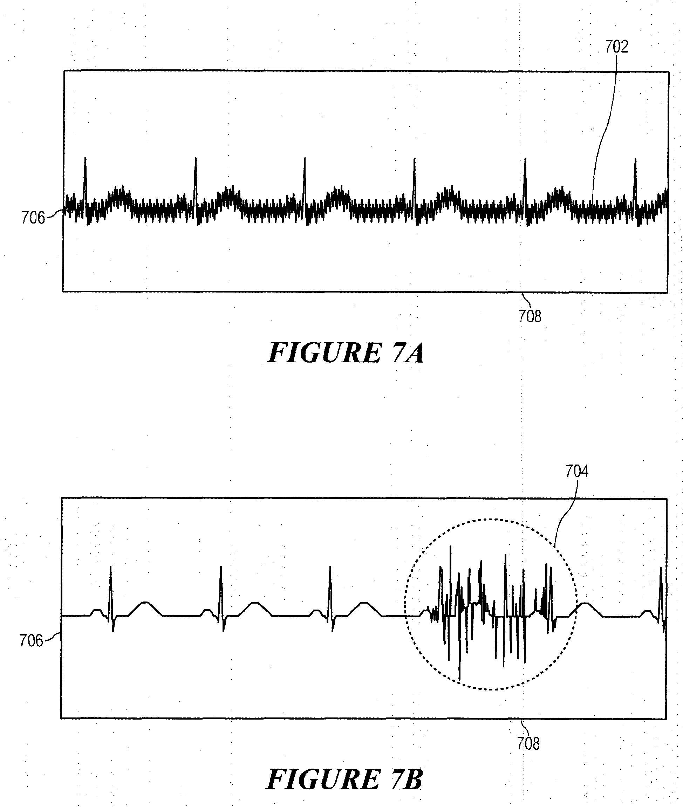

Figure 7A shows noise 702 typically present for a conventional 12 electrode wired system that is used to measure an ECG signal, the noise 702 arising from using an AC supply to power the conventional system. The vertical axis 706 represents voltage, while the horizontal axis 708 represents time.

In the ECG monitoring device 110 shown in Figures 2A to 3B, the noise 702 from using an AC supply is not an issue, since the ECG monitoring device 110 runs on a portable DC battery supply.

Comparing the ECG monitoring device 110 against standard medical test conditions, where 12 electrodes are used to detect an ECG signal, the ECG monitoring device 110 (by using only three electrodes 104A, 104B and 104C) provides a reduction in electrode spacing and a reduction in the number of electrodes used. This increases interference levels and reduces the ability to pick up a heart signal. In addition to this interference, noise 704 (see Figure 7B) arising from muscular tremors, is also present in an ECG signal. Such noise 704 is exacerbated by prolonged usage and usage under strenuous exercises that lead to perspiration.

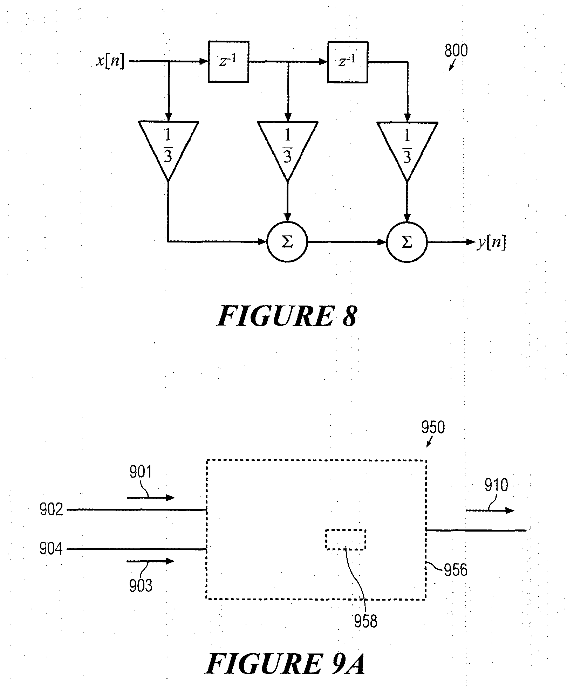

Figure 8 is a block diagram of a Finite Impulse Response (FIR) filter 800 to address the interference levels and the noise 704. In the embodiment shown in Figure 8, the FIR filter 800 is a second order/3-tap filter, implementing a moving average.

Figure 9A shows a schematic representation of circuitry 950 that can be used to address interference levels and also noise within an ECG signal. The circuitry 900 includes a primary input 902 configured to receive a first ECG signal 901 and a reference input 904 configured to receive a second ECG signal 903.

An adaptive filter 956, having a transfer function, is coupled to the primary input 902 and the reference input 904, wherein the transfer function self-adjusts according to the first ECG signal 901 and the second ECG signal 903 to produce co-efficients 958 for the adaptive filter 956 that reduce noise within the first ECG signal 901 and the second ECG signal 903, to output an ECG signal 910 having reduced noise.

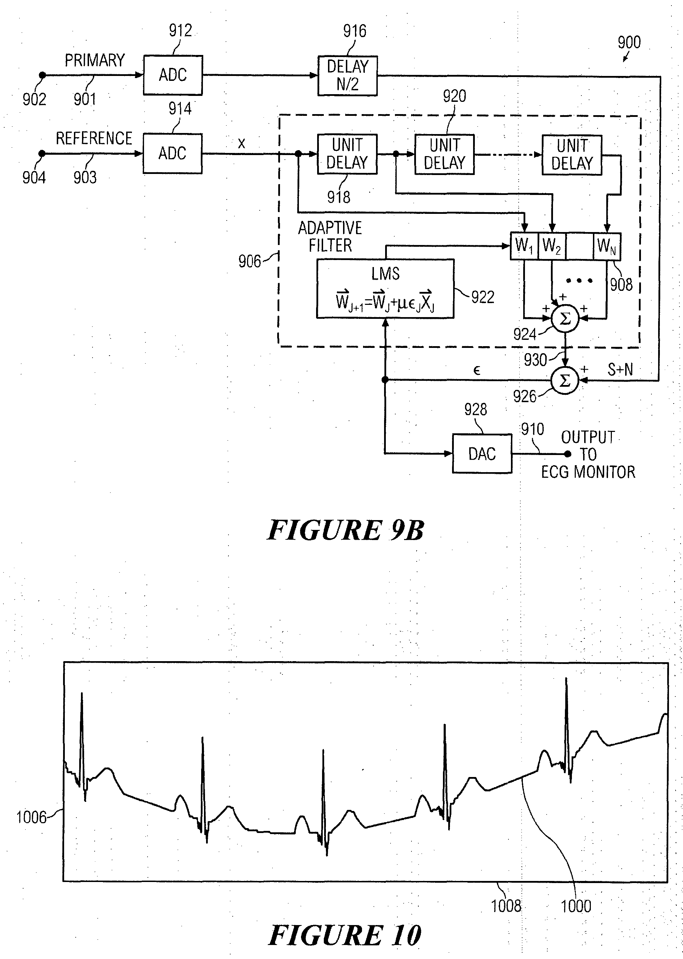

Figure 9B is a block diagram of a circuitry 900 implementing the schematic shown in Figure 9A. The circuitry 900 includes the primary input 902 configured to receive the first ECG signal 901 and the reference input 904 configured to receive the second ECG signal 903. In one embodiment, the circuitry 900 provides a digital filter, which may be used as an alternative to the filter described with reference to Figure 6 above.

An adaptive filter 906, having a transfer function, serves to process the first ECG signal 901 and the second ECG signal 903.

The circuitry 900 may be software based and includes the following: ADC (analogue to digital converter) blocks (912 and 914); delay blocks (916, 918 and 920), an adaptive filter coefficient block 908, a least mean square block 922, summing blocks (924 and 926) and a DAC (digital to analogue converter) block 928. These blocks are connected as follows.

A first ADC block 912 is connected to the primary input 902; and a second ADC block 914 is connected to the reference input 904. A first delay block 916 is connected to the first ADC block 912 and to a first summing block 926. The adaptive filter 906 is coupled to the second ADC block 914 and the first summing block 926. The DAC block 928 (from which the de-noised ECG signal 910 is output) is coupled to the first summing block 926 to receive the output from the first summing block 926.

Within the adaptive filter 906, a second delay block 918 is coupled to the second ADC block 914, the least mean square block 922 coupled to the first summing block 926, a second summing block 924 is coupled to the first summing block 926 and the adaptive filter coefficient block 908 is connected to the second ADC block 914. The adaptive filter coefficient block 908 is also connected to a delay block array 920, the least mean square block 922 and the second summing block 924. The delay block array 920 is connected to the second delay block 918.

In the embodiment shown in Figure 9B, noise is removed through deriving coefficients of the adaptive filter 906 from the first ECG signal 901, using the derived coefficients to process the second ECG signal 903 to produce an output and deducting the output (of the second ECG signal 903 having been processed with coefficients derived from the first ECG signal 901) from the first ECG signal 901.

Each co-efficient (w₁, w₂,... wₙ) generated by the adaptive filter coefficient block 908 is derived from processing the first ECG signal 901 or/and processing the second ECG signal 903 in one or more the delay blocks (916, 918 and 920). The co-efficients (w₁, w₂,... wₙ) are derived from
i) digitizing the first ECG signal 901 using the first ADC block 912;
ii) processing the digitized first ECG signal using the first delay block 916;
iii) summing, using the first summing block 926, the output from the first delay block 916 (being the digitized first ECG signal processed with delay) with output from the second summing block 924, wherein the output from the second summing block 924 is the result of the convolution of the co-efficients stored in the adaptive filter coefficient block 908 with the delayed ECG signals;
iv) performing a least mean square operation, using the least mean square block 922, on the output of the first summing block 926; and
v) using the output from the least mean square block 922 to change each co-efficient (w₁, w₂,... wₙ) of the adaptive filter coefficient block 908.

The various co-efficients (w₁, w₂,... wₙ) are used to modulate the second ECG signal 903 as follows.

The second ECG signal 903 is digitized by the second ADC block 914. The digitized second ECG signal is then multiplied by the co-efficient w₁. The co-efficient w₂ is multiplied by the digitized second ECG signal after having been processed by the second delay block 918. The co-efficient wₙ is multiplied by the digitized second ECG signal after having been processed by the second delay block 918 and one or more of the delay blocks, for example n-1 delay blocks, in the delay block array 920, for example for all integer n greater than or equal to 1 and less than or equal to N.

The output of the second summing block 924 is the result of the convolution of the co-efficients (w₁, w₂,... wₙ) stored in the adaptive filter coefficient block 908 with the delayed ECG signals. The output of the second summing block 924 is representative of noise signal 930 present in the first ECG signal 901. Since at the first summing block 926, the noise signal 930 is subtracted from the first ECG signal 901 (after having been processed by the first ADC block 912 and the first delay block 916), the output of the first summing block 926 is thus the denoised ECG signal 910.

In the following, baseline wandering reduction according to various embodiments will be described.

Figure 10 is a plot of voltage 1006 against time 1008, showing a signal 1000 where baseline wandering is present. Baseline wandering is exacerbated by prolonged usage and usage under strenuous exercises that lead to perspiration.

Designing an adaptive algorithm that corrects baseline wandering is challenging as skin impedance varies greatly between users. Even with proper skin preparation, there will be a delay in receiving a stable trace for a user with high skin impedance.

Various embodiments use an efficient recursive filter that estimates the state of a linear dynamic system from a series of noisy measurements. In an embodiment, a Kalman filter design is used. While cubic spline technique may also be used, the Kalman filter design shows better performance.

In the following, QRS complex reproduction according to various embodiments will be described.

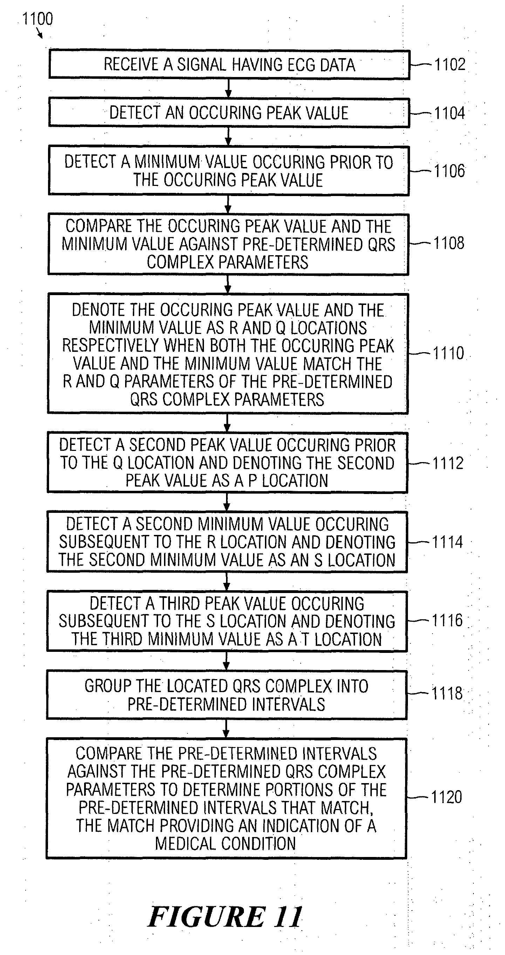

Figure 11 shows a flow chart for a method 1100, according to an embodiment, to locate a QRS complex within ECG data. In an embodiment, the method 1100 is used to process ECG signals detected by the ECG monitoring device 110 (see Figure ID).

At 1102, a signal having ECG data is received. At 1104, a first occurring peak value is detected. At 1106, a first minimum value occurring prior to the first occurring peak value is detected. At 1108, the first occurring peak value and the minimum value is compared against pre-determined QRS complex parameters, such as, for example, pre-set cut-offs with QRS duration greater than 70ms and QRS amplitude greater than 250uV. At 1110, the first occurring peak value and the minimum value are denoted as R and Q locations respectively when both the first occurring peak value and the minimum value match the R and Q parameters of the pre-determined QRS complex parameters.

At 1110, the minimum value is denoted as the Q location only if the minimum value occurs within a pre-defined interval from the peak value.

At 1104 and 1106, any one or more of the first occurring peak and the first minimum value are detected by processing the signal having the ECG data using a differential equation. The differential equation may be a first order differentiation, followed by second order differentiation. In an embodiment, a first derivative is obtained for the ECG signal, where the first derivative is set to zero and the resulting equation is solved to locate turning points within the ECG signal. A second derivative is then calculated to determine whether the turning point is a peak value or a minimum value.

At 1112, a second peak value occurring prior to the Q location is detected, whereby the second peak value is denoted as a P location. At 1114, a second minimum value occurring subsequent to the R location is detected, whereby the second minimum value is denoted as an S location. At 1116, a third peak value occurring subsequent to the S location is detected, whereby the third peak value is denoted as a T location.

At 1112, 1114 and 1116, the second peak value, the second minimum value and the third peak value are respectively denoted as the P, S and T locations only if the second peak value, the second minimum value and the third peak value occur within a pre-defined interval from the R or Q location.

Any one or more of the second peak value, the second minimum value and the third peak value are detected by processing the signal having the ECG data using a differential equation. The differential equation may be a first order differentiation, followed by second order differentiation.

A portion of the method 1100 may be reiterated, whereby 1104 to 1110 is repeated as follows. At 1104, a peak value occurring subsequent to an earlier peak value is detected. At 1106, a minimum value occurring prior to the peak value is detected. At 1108, the peak value and the minimum value are compared against pre-determined QRS complex parameters. At 1110, the peak value and the minimum value are denoted as subsequent R and Q locations respectively when both the peak value and the minimum value match the R and Q parameters of the pre-determined QRS complex parameters.

At 1118, the located QRS complex is grouped into pre-determined intervals. At 1120, the pre-determined intervals are compared against the pre-determined QRS complex parameters to determine portions of the pre-determined intervals that match, the match providing an indication of a medical condition. The pre-determined interval may be a multiple of 10 seconds.

At 1108, comparing the first occurring peak value and the minimum value against pre-determined QRS complex parameters may be conducted using one or more of the following: an image processing technique, heuristic determination or using an artificial neural learning network.

At 1108, the first occurring peak value and the minimum value may be matched against any one or more of the pre-determined QRS complex parameters comprising: minimum QRS amplitude, QRS interval, PR interval, PR range, QT interval, constancy of PR interval and constancy of RR interval.

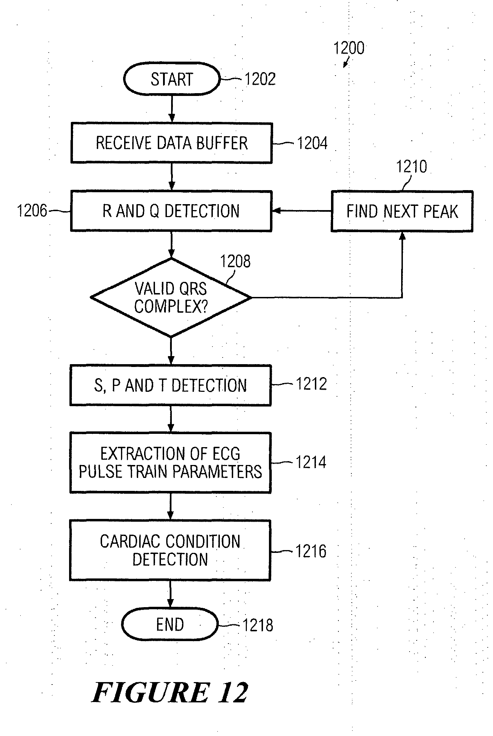

Figure 12 shows a flow chart for an algorithm 1200 implementing the method 1100 of Figure 11.

The algorithm 1200 starts at 1202, where ECG data is received at 1204. At 1206, R and Q locations are detected so as to form a valid QRS complex at 1208. A loop occurs for 1206 and 1208, i.e. other R and Q values are located to form further QRS complexes, through locating subsequent peak values at 1210. For 1204 to 1210, the algorithm 1200 works in a similar manner as described for 1102 to 1110 of Figure 11 above and is thus not further described.

At 1212, S, P and T locations are detected, in a manner similar as that described in 1112 to 1116 of Figure 11 above. At 1214, the located PQRST values (see Figure 13 for an exemplary PQRST complex 1302, in a graph of a plot of voltage 1006 against time 1008) are used to generate an ECG pulse train so that a cardiac condition may be detected at 1216. The formation of the ECG pulse train at 1214 and the detection of the cardiac condition at 1216 is similar to 1118 and 1120 described above and is thus not further described. At 1218, the algorithm 1200 ends.

The collected ECG waveforms may be compared to pre-determined parameters for pattern recognition and rhythm determination that may include the use of image processing technique, heuristic determination and an artificial neural learning network. Besides relying on human interpretation, regularity or irregularity of the electrical signals of the heart may be determined by computer intelligent algorithms in real time based on pattern recognition algorithm and rhythm determination parameters. The collected ECG data may also be used to extract physiological indicators that include, but are not limited to, heart rate, basal metabolic rate, VO2max (maximum volume of oxygen that can be utilized in one minute during maximal or exhaustive exercise), heart rate recovery, heart rate variability and calories consumption. These physiological indicators may be displayed on an LCD display of a receiver or a computer display, both in wireless communication with the ECG monitoring device 110 (see Figure 1D).

Figure 14 shows a network 1400 where an electrocardiographic monitoring system, according to an embodiment, is implemented.

The ECG monitoring device 110 is worn by a user to monitor a physical activity performed by the user. The ECG monitoring device 110 may communicate with a mobile device 1404 or a web server 1406, both through a base station 1402. While Figure 14 shows that the mobile device 1404 is a mobile phone, any one or more of the following devices may be used: a PDA, a tablet and a laptop. The web server 1406 may be connected to a PC 1408. It is also possible for the ECG monitoring device 110 to transmit ECG signals directly to a computer via a wireless communications protocol. The ECG monitoring device 110 may allow a user to discover various irregular symptoms from a detected PQRST signal, such as hypertrophy, hyperkelemia, ischemia, and infarction.

Although not shown in Figure 14, the ECG monitoring device 110 may transmit processed ECG signal, via a wireless platform of available technologies, to a preferred mobile receiver for ambulatory needs. The wireless transmission platform may be a Bluetooth module on the electrical circuit board of the ECG monitoring device 110. Other wirelesss transmission platforms include, but not are not limited to, infra-red, WiFi, or low powered radio frequency (RF) which are common communication protocol to many data receiving tools.

One advantage of the ECG monitoring device 110 is that non-intrusive and yet accurate monitoring for time periods longer than a few hours may be provided. The ECG monitoring device 110 can be fitted with onboard storage capability (such as Transflash or Nand flash) and GPRS or other transmission capabilities to act as a 24-hour continuous operating standalone device that can transmit to a computer server.

Through analysis of long-term data trends collected, a more accurate prognosis can be made on physiological conditions that include, but are not limited to, the heart either under normal condition or diseased condition of either naturally occurring or pharmocological drug-induced. The electrocardiographic monitoring system is wireless, un-obstructive and provides continuous and real time monitoring of physiological functions that seemlessly integrates in the daily life of a user. With capturing of real time information over a long period of time, intensive management of an ongoing disease through pharmacological means or pre-emptive interventions of a developing disease (that may or may not be symptomatic to the patients or users) is facilitated. The ECG monitoring device 110 may also have additional sensors for monitoring of other important physiological conditions such as, but not limited to, glucose levels, blood pressure, body temperature and hydration levels.

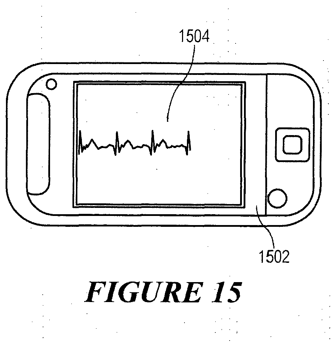

Figure 15 shows a light and mobile telecommunications tool 1502 that has a LCD display 1504 for real time viewing of an ECG signal streaming from the ECG monitoring device 110 (see Figure 14). However, other data receiving tools, that may or may not have viewer display, may be used.

The received data may be processed with noise cancelling and filtering techniques not restricted to a Finite Impulse Response (FIR) filter (as described with reference to Figure 8) and Kalman filter to minimize motion artifacts and baseline wandering of the ECG signal. The telecommunications tool 1502 may also act as a data storage/data display and relaying station through GPRS platform to a central computer server for in-depth data analysis and tracking of data trends. The transmission of the data from the telecommunications tool 1502 to a central computer server may be performed using EDGE, 3G, HSDPA, LTE, WiFi, WiMax or cable. The telecommunications tool 1502 may also transmit part or whole of the ECG data to another mobile telecommunications tool such as mobile phone for data review.

While embodiments of the invention will be shown and described with reference to specific embodiments, it should be understood by those skilled in the art that various changes in form and detail may be made therein without departing from the scope of the invention as defined by the appended claims.

## Claims

1. An electrocardiographic monitoring system (100, 101) comprising:
a housing (102, 152) configured to be attached to a creature body part (140), the housing (102, 152) comprising:
a plurality of electrodes (104, 104A, 104B, 104C, 154, 154A, 154B and 154C) confined within the boundary of the housing (102, 152), the plurality of electrodes (104, 104A, 104B, 104C, 154, 154A, 154B and 154C) arranged a distance apart (304, 306 and 308) from each other and accessible from a same exterior surface (102s, 152s) of the housing (102, 152);
a fastening mechanism (186, 430) provided on each electrode (104, 104A, 104B, 104C, 154, 154A, 154B and 154C) of the plurality of electrodes (104, 104A, 104B, 104C, 154, 154A, 154B and 154C) to allow detachable fastening between the electrode (104, 104A, 104B, 104C, 154, 154A, 154B and 154C) and an electrode lead;
a signal processor (106, 156) configured to receive electrocardiogram (ECG) signals from any one or more of the plurality of electrodes (104, 104A, 104B, 104C, 154, 154A, 154B and 154C) and transmit signals to any one or more of the plurality of electrodes (104, 104A, 104B, 104C, 154, 154A, 154B and 154C); and
a transmitter (108, 158) configured to transmit signals from the signal processor (106, 156),
wherein the electrodes (104, 104A, 104B, 104C, 154, 154A, 154B and 154C) are preferably arranged along a straight line,
wherein the signal processor (106, 156) is further configured to locate a plurality of QRS complexes in the ECG signals received, **characterized in that**:
the signal processor (106, 156) is
further configured to:
detect a first occurring peak value in the ECG signals;
detect a first minimum value in the ECG signals occurring prior to the first occurring peak value;
compare the first occurring peak value and the first minimum value against pre-determined QRS complex parameters;
denote the first occurring peak value and the first minimum value as R and Q locations respectively when both the first occurring peak value and the first minimum value match the R and Q parameters of the pre-determined QRS complex parameters to form a first QRS complex of the plurality of QRS complexes;
detect a second peak occurring prior to the Q location and denote the second peak as a P location;
detect a second minimum value occurring subsequent to the R location and denote the second minimum value as an S location; and
detect a third peak value occurring subsequent to the S location and denote the third peak value as a T location, wherein the second peak value, the second minimum value and the third peak value are respectively denoted as the P, S and T locations only if the second peak value, the second minimum value and the third peak value occur within a pre-defined interval from the R or Q location;
reiterate the process of:
detecting a further peak value occurring subsequent to an earlier peak value;
detecting a further minimum value occurring prior to the further peak value;
comparing the further peak value and the further minimum value against pre-determined QRS complex parameters; and
denoting the further peak value and the further minimum value as subsequent R and Q locations respectively when both the further peak value and the further minimum value match the R and Q parameters of the pre-determined QRS complex parameters to form a further QRS complex of the plurality of QRS complexes;
wherein the first occurring peak value and the minimum value are matched against any one or more of the pre-determined QRS complex parameters comprising: minimum QRS amplitude, QRS interval, PR interval, PR range, QT interval, constancy of PR interval, and constancy of RR interval; and
wherein the signal processor (106, 156) comprises:
a differential input buffer (156dib, 602) coupled to at least two input electrodes of the plurality of electrodes (104, 104A, 104B, 104C, 154, 154A, 154B and 154C);
a subtractor circuit (156sc, 608) coupled to the differential input buffer (156dib, 602);
an amplifier (156a, 610) coupled to the subtractor circuit (156sc, 608);
a baseline restoration circuit (156brc, 612) coupled to the amplifier (156a, 610) and the subtractor circuit (156sc, 608); and
a voltage bias circuit (156vbc, 616) coupled to the amplifier (156a, 610) and the baseline restoration circuit (156brc, 612),
wherein the subtractor circuit (156sc, 608), the voltage bias circuit (156vbc, 616) and the baseline restoration circuit (156brc, 612) are configured to maintain a baseline of the ECG signals,
wherein:
the differential input buffer (602) comprises first and second high-impedance amplifiers configured to monitor a voltage between an input and an interface ground;
a first port (604) of the differential input buffer (602) coupled to a first input electrode (104A) of the at least two input electrodes provides an input for one of the first and second high-impedance amplifiers, while a second port (606) of the differential input buffer (602) coupled to a second input electrode (104B) of the at least two input electrodes provides an input for the other of the first and second high-impedance amplifiers;
the subtractor circuit (608) comprises a third amplifier configured to subtract the outputs of the two high-impedance amplifiers to remove any signal common to both the input signal at the first port (604) and the input signal at the second port (606);
the signal difference between the input signal at the first port (604) and the input signal at the second port (606) forms a baseline signal (614), which is sent to both the amplifier (610) and the baseline restoration circuit (612);
the baseline restoration circuit (612) is configured to compare the baseline signal (614) and a biasing voltage signal (628) from the voltage bias circuit (616) and to feed back a resulting correction signal (626) to the subtractor circuit (608); and
the biasing voltage signal (628) is used to correct baseline swings in the baseline signal (614).

2. The electrocardiographic monitoring system (100, 101) according to claim 1, wherein the plurality of electrodes (104, 104A, 104B, 104C, 154, 154A, 154B and 154C) comprise at least three input electrodes (154A, 154B and 154C) configured to send signals to the signal processor (106, 156).

3. The electrocardiographic monitoring system (100, 101) according to claim 1 or 2, wherein the plurality of electrodes (104, 104A, 104B, 104C, 154, 154A, 154B and 154C) comprise an electrode (154C) configured as a ground terminal.

4. The electrocardiographic monitoring system (100, 101) according to any one of the preceding claims,
wherein the signal processor (106, 156) further comprises a low pass filter (1561pf, 620) coupled to the amplifier (156a, 610) and the voltage bias circuit (156vbc, 616), and/or a neutral circuit (156nc) coupled to the voltage bias circuit (156vbc, 616), the neutral circuit (156nc, 618) coupled to a ground terminal electrode (154C) of the plurality of electrodes (104, 104A, 104B, 104C, 154, 154A, 154B and 154C).

5. The electrocardiographic monitoring system (100, 101) according to claim 4,
wherein the signal processor (106, 156) further comprises an analogue to digital converter (156adc, 622) coupled to the low pass filter (1561pf, 620) and the transmitter (108, 158).

6. The electrocardiographic monitoring system (100, 101) according to any one of the preceding claims, wherein the distance between each centre of the plurality of electrodes (104, 104A, 104B, 104C, 154, 154A, 154B and 154C) is less than 5cm.

7. The electrocardiographic monitoring system (100, 101) according to any one of the preceding claims, wherein the shape of the housing (102, 152) has a longest length (202) of less than 7cm.

8. The electrocardiographic monitoring system (100, 101) according to any one of the preceding claims, wherein an equal distance (304, 306 and 308) exists between each centre of the plurality of electrodes (104, 104A, 104B, 104C, 154, 154A, 154B and 154C).

9. The electrocardiographic monitoring system (100, 101) according to any one of the preceding claims, wherein the plurality of electrodes (104, 104A, 104B, 104C, 154, 154A, 154B and 154C) are arranged so that each electrode (104, 104A, 104B, 104C, 154, 154A, 154B and 154C) is located at a vertice of a triangle,
wherein the triangle is preferably equilateral or isosceles.

10. The electrocardiographic monitoring system (100, 101) according to any one of the preceding claims, further comprising:
a receiver (112, 162) to receive signals from the transmitter (108, 158);
a processor (116, 166) coupled to the receiver (112, 162), the processor (116, 166) configured to process the signals received from the transmitter (108, 158); and
a display (118, 168) coupled to the processor (116, 166), the display (118, 168) configured to show ECG data from the signals received by the receiver (112, 162).

11. The electrocardiographic monitoring system (100, 101) according to claim 10, wherein the receiver (112, 162), the processor (116, 166) and the display (118, 168) are located in a platform (120, 170) remote from the housing (102, 152).

12. The electrocardiographic monitoring system (100, 101) according to claim 11, wherein the platform (120, 170) comprises any one or more of the following devices: a computer server (1406), a mobile device (1404) and a computer terminal (1408).

13. The electrocardiographic monitoring system (100, 101) according to claim 12, wherein the mobile device (1404) comprises any one or more of the following devices: a mobile phone, a PDA, a tablet and a laptop.

14. The electrocardiographic monitoring system (100, 101) according to any one of the preceding claims, further comprising a power source (115, 165) to power the signal processor (106, 156) and the transmitter (108, 158).

15. The electrocardiographic monitoring system (100, 101) according to any one of the preceding claims, wherein the transmitter (108, 158) transmits signals using any one or more of the following mediums: EDGE, bluetooth, 3G, HSDPA, LTE, WiFi, WiMax, low power RF operating in ISM band or cable.

## Patentansprüche

1. Elektrokardiographisches Überwachungssystem (100, 101), aufweisend:
ein Gehäuse (102, 152), das so konfiguriert ist, dass es an einem Körperteil (140) eines Lebewesens angebracht werden kann, wobei das Gehäuse (102, 152) aufweist:
eine Mehrzahl von Elektroden (104, 104A, 104B, 104C, 154, 154A, 154B und 154C), die innerhalb der Begrenzung des Gehäuses (102, 152) eingeschlossen sind, wobei die Mehrzahl von Elektroden (104, 104A, 104B, 104C, 154, 154A, 154B und 154C) in einem Abstand (304, 306 und 308) voneinander angeordnet und von einer gleichen Außenfläche (102s, 152s) des Gehäuses (102, 152) zugänglich sind;
einen Befestigungsmechanismus (186, 430), der an jeder Elektrode (104, 104A, 104B, 104C, 154, 154A, 154B und 154C) der Mehrzahl von Elektroden (104, 104A, 104B, 104C, 154, 154A, 154B und 154C) bereitgestellt ist, um eine lösbare Befestigung zwischen der Elektrode (104, 104A, 104B, 104C, 154, 154A, 154B und 154C) und einer Elektrodenleitung zu ermöglichen;
einen Signalprozessor (106, 156), der so konfiguriert ist, dass er Elektrokardiogramm-(EKG)-Signale von einer oder mehreren der Mehrzahl von Elektroden (104, 104A, 104B, 104C, 154, 154A, 154B und 154C) empfängt und Signale an eine oder mehrere der Mehrzahl von Elektroden (104, 104A, 104B, 104C, 154, 154A, 154B und 154C) sendet; und
einen Sender (108, 158), der so konfiguriert ist, dass er Signale von dem Signalprozessor (106, 156) überträgt,
wobei die Elektroden (104, 104A, 104B, 104C, 154, 154A, 154B und 154C) vorzugsweise entlang einer geraden Linie angeordnet sind,
wobei der Signalprozessor (106, 156) weiterhin konfiguriert ist, um eine Mehrzahl von QRS-Komplexen in den empfangenen EKG-Signalen zu lokalisieren,
**dadurch gekennzeichnet, dass**:
der Signalprozessor (106, 156) weiterhin konfiguriert ist, um
einen ersten auftretenden Spitzenwert in den EKG-Signalen zu erfassen;
einen ersten Minimalwert in den EKG-Signalen zu erfassen, der vor dem ersten auftretenden Spitzenwert auftritt;
den ersten auftretenden Spitzenwert und den ersten Minimalwert mit vorbestimmten QRS-Komplex-Parametern zu vergleichen;
den ersten auftretenden Spitzenwert und den ersten Minimalwert als R-bzw. Q-Stellen zu bezeichnen, wenn sowohl der erste auftretende Spitzenwert als auch der erste Minimalwert mit den R- und Q-Parametern der vorbestimmten QRS-Komplex-Parameter übereinstimmen, um einen ersten QRS-Komplex der Mehrzahl von QRS-Komplexen zu bilden;
einen zweiten Spitzenwert, der vor der Q-Stelle auftritt, zu erfassen und den zweiten Spitzenwert als P-Stelle zu bezeichnen;
einen zweiten Minimalwert, der nach der R-Stelle auftritt, zu erfassen und den zweiten Minimalwert als S-Stelle zu bezeichnen; und
einen dritten Spitzenwert, der nach der S-Stelle auftritt, zu erfassen und den dritten Spitzenwert als T-Stelle zu bezeichnen, wobei der zweite Spitzenwert, der zweite Minimalwert und der dritte Spitzenwert nur dann als P-, S- bzw. T-Stelle bezeichnet werden, wenn der zweite Spitzenwert, der zweite Minimalwert und der dritte Spitzenwert innerhalb eines vordefinierten Intervalls ab der R- oder Q-Stelle auftreten;
den Prozesses des:
Erfassens eines weiteren Spitzenwertes, der nach einem früheren Spitzenwert auftritt,
Erfassens eines weiteren Minimalwerts, der vor dem weiteren Spitzenwert auftritt,
Vergleichens des weiteren Spitzenwerts und des weiteren Minimalwerts mit vorbestimmten QRS-Komplex-Parametern, und
Kennzeichnens des weiteren Spitzenwertes und des weiteren Minimalwertes als nachfolgende R- bzw. Q-Stellen, wenn sowohl der weitere Spitzenwert als auch der weitere Minimalwert mit den R- und Q-Parametern der vorbestimmten QRS-Komplex-Parameter übereinstimmen, um einen weiteren QRS-Komplex der Mehrzahl von QRS-Komplexen zu bilden,
zu wiederholen;
wobei der erste auftretende Spitzenwert und der Minimalwert mit einem oder mehreren der vorbestimmten QRS-Komplex-Parameter abgeglichen werden, die umfassen: minimale QRS-Amplitude, QRS-Intervall, PR-Intervall, PR-Bereich, QT-Intervall, Konstanz des PR-Intervalls und Konstanz des RR-Intervalls; und
wobei der Signalprozessor (106, 156) aufweist:
einen Differenzeingangspuffer (156dib, 602), der mit mindestens zwei Eingangselektroden der Mehrzahl von Elektroden (104, 104A, 104B, 104C, 154, 154A, 154B und 154C) gekoppelt ist;
eine Subtrahierschaltung (156sc, 608), die mit dem Differenzeingangspuffer (156dib, 602) gekoppelt ist;
einen Verstärker (156a, 610), der mit der Subtrahierschaltung (156sc, 608) gekoppelt ist;
eine Grundlinienwiederherstellungsschaltung (156brc, 612), die mit dem Verstärker (156a, 610) und der Subtrahierschaltung (156sc, 608) gekoppelt ist; und
eine Vorspannungsschaltung (156vbc, 616), die mit dem Verstärker (156a, 610) und der Grundlinienwiederherstellungsschaltung (156brc, 612) gekoppelt ist,
wobei die Subtrahierschaltung (156sc, 608), die Vorspannungsschaltung (156vbc, 616) und die Grundlinienwiederherstellungsschaltung (156brc, 612) so konfiguriert sind, dass sie eine Grundlinie der EKG-Signale aufrechterhalten,
wobei:
der Differenzeingangspuffer (602) einen ersten und einen zweiten Hochimpedanzverstärker aufweist, die so konfiguriert sind, dass sie eine Spannung zwischen einem Eingang und einer Schnittstellenmasse überwachen;
ein erster Anschluss (604) des Differenzeingangspuffers (602), der mit einer ersten Eingangselektrode (104A) der mindestens zwei Eingangselektroden gekoppelt ist, einen Eingang für einen des ersten und zweiten Hochimpedanzverstärkers bereitstellt, und ein zweiter Anschluss (606) des Differenzeingangspuffers (602), der mit einer zweiten Eingangselektrode (104B) der mindestens zwei Eingangselektroden gekoppelt ist, einen Eingang für den anderen des ersten und zweiten Hochimpedanzverstärkers bereitstellt;
die Subtrahierschaltung (608) einen dritten Verstärker aufweist, der so konfiguriert ist, dass er die Ausgaben der beiden Hochimpedanzverstärker subtrahiert, um jegliches Signal zu entfernen, das sowohl dem Eingangssignal am ersten Anschluss (604) als auch dem Eingangssignal am zweiten Anschluss (606) gemeinsam ist;
die Signaldifferenz zwischen dem Eingangssignal am ersten Anschluss (604) und dem Eingangssignal am zweiten Anschluss (606) ein Grundliniensignal (614) bildet, das sowohl an den Verstärker (610) als auch an die Grundlinienwiederherstellungsschaltung (612) gesendet wird;
die Grundlinienwiederherstellungsschaltung (612) so konfiguriert ist, dass sie das Grundliniensignal (614) und ein Vorspannungssignal (628) von der Vorspannungsschaltung (616) vergleicht und ein resultierendes Korrektursignal (626) an die Subtrahierschaltung (608) zurückführt; und
das Vorspannungssignal (628) verwendet wird, um Grundlinienschwankungen in dem Grundliniensignal (614) zu korrigieren.

2. Elektrokardiographisches Überwachungssystem (100, 101) nach Anspruch 1, wobei die Mehrzahl von Elektroden (104, 104A, 104B, 104C, 154, 154A, 154B und 154C) mindestens drei Eingangselektroden (154A, 154B und 154C) aufweist, die so konfiguriert sind, dass sie Signale an den Signalprozessor (106, 156) senden.

3. Elektrokardiographisches Überwachungssystem (100, 101) nach Anspruch 1 oder 2, wobei die Mehrzahl von Elektroden (104, 104A, 104B, 104C, 154, 154A, 154B und 154C) eine Elektrode (154C) aufweist, die als Masseanschluss konfiguriert ist.

4. Elektrokardiographisches Überwachungssystem (100, 101) nach einem der vorhergehenden Ansprüche,
wobei der Signalprozessor (106, 156) ferner ein Tiefpassfilter (1561pf, 620) aufweist, das mit dem Verstärker (156a, 610) und der Vorspannungsschaltung (156vbc, 616) gekoppelt ist, und/oder eine Neutralschaltung (156nc), die mit der Vorspannungsschaltung (156vbc, 616) gekoppelt ist, wobei die Neutralschaltung (156nc, 618) mit einer Erdungsanschlusselektrode (154C) der Mehrzahl von Elektroden (104, 104A, 104B, 104C, 154, 154A, 154B und 154C) gekoppelt ist.

5. Elektrokardiographisches Überwachungssystem (100, 101) nach Anspruch 4,
wobei der Signalprozessor (106, 156) ferner einen Analog-Digital-Wandler (156adc, 622) aufweist, der mit dem Tiefpassfilter (1561pf, 620) und dem Sender (108, 158) gekoppelt ist.

6. Elektrokardiographisches Überwachungssystem (100, 101) nach einem der vorhergehenden Ansprüche, wobei der Abstand zwischen jeder Mitte der Mehrzahl von Elektroden (104, 104A, 104B, 104C, 154, 154A, 154B und 154C) weniger als 5 cm beträgt.

7. Elektrokardiographisches Überwachungssystem (100, 101) nach einem der vorhergehenden Ansprüche, wobei die Form des Gehäuses (102, 152) eine längste Länge (202) von weniger als 7 cm aufweist.

8. Elektrokardiographisches Überwachungssystem (100, 101) nach einem der vorhergehenden Ansprüche, wobei ein gleicher Abstand (304, 306 und 308) zwischen jeder Mitte der Mehrzahl von Elektroden (104, 104A, 104B, 104C, 154, 154A, 154B und 154C) besteht.

9. Elektrokardiographisches Überwachungssystem (100, 101) nach einem der vorhergehenden Ansprüche, wobei die Mehrzahl von Elektroden (104, 104A, 104B, 104C, 154, 154A, 154B und 154C) so angeordnet sind, dass jede Elektrode (104, 104A, 104B, 104C, 154, 154A, 154B und 154C) an einer Ecke eines Dreiecks angeordnet ist,
wobei das Dreieck vorzugsweise gleichseitig oder gleichschenklig ist.

10. Elektrokardiographisches Überwachungssystem (100, 101) nach einem der vorhergehenden Ansprüche, ferner aufweisend:
einen Empfänger (112, 162) zum Empfangen von Signalen von dem Sender (108, 158);
einen Prozessor (116, 166), der mit dem Empfänger (112, 162) gekoppelt ist, wobei der Prozessor (116, 166) konfiguriert ist, um die von dem Sender (108, 158) empfangenen Signale zu verarbeiten; und
eine Anzeige (118, 168), die mit dem Prozessor (116, 166) gekoppelt ist, wobei die Anzeige (118, 168) so konfiguriert ist, dass sie EKG-Daten aus den von dem Empfänger (112, 162) empfangenen Signalen anzeigt.

11. Elektrokardiographisches Überwachungssystem (100, 101) nach Anspruch 10, wobei der Empfänger (112, 162), der Prozessor (116, 166) und die Anzeige (118, 168) in einer Plattform (120, 170) entfernt vom Gehäuse (102, 152) angeordnet sind.

12. Elektrokardiographisches Überwachungssystem (100, 101) nach Anspruch 11, wobei die Plattform (120, 170) eine oder mehrere der folgenden Vorrichtungen umfasst: einen Computerserver (1406), eine mobile Vorrichtung (1404) und ein Computerterminal (1408).

13. Elektrokardiographisches Überwachungssystem (100, 101) nach Anspruch 12, wobei die mobile Vorrichtung (1404) eine oder mehrere der folgenden Vorrichtungen umfasst: ein Mobiltelefon, einen PDA, ein Tablet und einen Laptop.

14. Elektrokardiographisches Überwachungssystem (100, 101) nach einem der vorhergehenden Ansprüche, ferner aufweisend eine Stromquelle (115, 165) zur Versorgung des Signalprozessors (106, 156) und des Senders (108, 158).

15. Elektrokardiographisches Überwachungssystem (100, 101) nach einem der vorangehenden Ansprüche, wobei der Sender (108, 158) Signale unter Verwendung eines oder mehrerer der folgenden Medien überträgt: EDGE, Bluetooth, 3G, HSDPA, LTE, WiFi, WiMax, RF mit geringer Leistung im ISM-Band arbeitend, oder Kabel.

## Revendications

1. Système de surveillance électrocardiographique (100, 101) comprenant :
un boîtier (102, 152) conçu pour être fixé à une partie du corps d'un être vivant (140), le boîtier (102, 152) comprenant :
une pluralité d'électrodes (104, 104A, 104B, 104C, 154, 154A, 154B et 154C) confinées dans la limite du boîtier (102, 152), la pluralité d'électrodes (104, 104A, 104B, 104C, 154, 154A, 154B et 154C) étant agencées espacées d'une certaine distance (304, 306 et 308) les unes des autres et étant accessibles depuis une même surface extérieure (102s, 152s) du boîtier (102, 152) ;
un mécanisme de fixation (186, 430) fourni sur chaque électrode (104, 104A, 104B, 104C, 154, 154A, 154B et 154C) de la pluralité d'électrodes (104, 104A, 104B, 104C, 154, 154A, 154B et 154C) pour permettre une fixation détachable entre l'électrode (104, 104A, 104B, 104C, 154, 154A, 154B et 154C) et un fil d'électrode ;
un processeur de signaux (106, 156) conçu pour recevoir des signaux d'électrocardiogramme (ECG) provenant d'une ou plusieurs électrodes quelconques de la pluralité d'électrodes (104, 104A, 104B, 104C, 154, 154A, 154B et 154C) et transmettre des signaux à une ou plusieurs électrodes quelconques de la pluralité d'électrodes (104, 104A, 104B, 104C, 154, 154A, 154B et 154C) ; et
un émetteur (108, 158) conçu pour émettre des signaux provenant du processeur de signaux (106, 156),
dans lequel les électrodes (104, 104A, 104B, 104C, 154, 154A, 154B et 154C) sont de préférence agencée le long d'une ligne droite,
dans lequel le processeur de signaux (106, 156) est en outre conçu pour localiser une pluralité de complexes QRS dans les signaux d'ECG reçus, **caractérisé en ce que** :
le processeur de signaux (106, 156) est en outre conçu pour :
détecter une première valeur de crête apparaissant dans les signaux d'ECG ;
détecter une première valeur minimale dans les signaux d'ECG survenant avant la première valeur de crête ;
comparer la première valeur de crête apparaissant et la première valeur minimale par rapport à des paramètres de complexe QRS prédéterminés ;
désigner la première valeur de crête et la première valeur minimale comme des emplacements R et Q respectivement lorsqu'à la fois la première valeur de crête et la première valeur minimale correspondent aux paramètres R et Q des paramètres de complexe QRS prédéterminés pour former un premier complexe QRS de la pluralité de complexes QRS ;
détecter une seconde crête survenant avant l'emplacement Q et désigner la seconde crête comme un emplacement P ;
détecter une seconde valeur minimale survenant après l'emplacement R et désigner la seconde valeur minimale comme un emplacement S ; et
détecter une troisième valeur de crête survenant après l'emplacement S et désigner la troisième valeur de crête comme un emplacement T, dans lequel la deuxième valeur de crête, la deuxième valeur minimale et la troisième valeur de crête sont désignées respectivement comme les emplacements P, S et T uniquement si la deuxième valeur de crête, la deuxième valeur minimale et la troisième valeur de crête surviennent dans un intervalle prédéfini par rapport à l'emplacement R ou Q;
réitérer le processus de :
détection d'une autre valeur de crête survenant après une valeur de crête antérieure ;
détection d'une autre valeur minimale survenant avant l'autre valeur de crête ;
comparaison de l'autre valeur de crête et de l'autre valeur minimale par rapport à des paramètres de complexe QRS prédéterminés ; et
désignation de l'autre valeur de crête et de l'autre valeur minimale comme les emplacements R et Q suivants respectivement lorsqu'à la fois l'autre valeur de crête et l'autre valeur minimale correspondent aux paramètres R et Q des paramètres de complexe QRS prédéterminés pour former un autre complexe QRS de la pluralité de complexes QRS ;
dans lequel la première valeur de crête et la valeur minimale sont comparées à un ou plusieurs paramètres quelconques des paramètres de complexe QRS prédéterminés comprenant :
l'amplitude de QRS minimale, l'intervalle QRS, l'intervalle PR, la plage PR, l'intervalle QT, la constance de l'intervalle PR, et la constance de l'intervalle RR ; et
dans lequel le processeur de signaux (106, 156) comprend :
un tampon d'entrée différentiel (156dib, 602) couplé à au moins deux électrodes d'entrée de la pluralité d'électrodes (104, 104A, 104B, 104C, 154, 154A, 154B et 154C) ;
un circuit soustracteur (156sc, 608) couplé au tampon d'entrée différentiel (156dib, 602) ;
un amplificateur (156a, 610) couplé au circuit soustracteur (156sc, 608) ;
un circuit de restauration de ligne de base (156brc, 612) couplé à l'amplificateur (156a, 610) et au circuit soustracteur (156sc, 608) ; et
un circuit de polarisation de tension (156vbc, 616) couplé à l'amplificateur (156a, 610) et au circuit de restauration de ligne de base (156brc, 612),
dans lequel le circuit soustracteur (156sc, 608), le circuit de polarisation de tension (156vbc, 616) et le circuit de restauration de ligne de base (156brc, 612) sont configurés pour maintenir une ligne de base des signaux d'ECG ;
dans lequel :
le tampon d'entrée différentiel (602) comprend des premier et deuxième amplificateurs à haute impédance configurés pour surveiller une tension entre une entrée et une terre d'interface ;
un premier port (604) du tampon d'entrée différentiel (602) couplé à une première électrode d'entrée (104A) des au moins deux électrodes d'entrée fournit une entrée pour un des premier et deuxième amplificateurs à haute impédance, tandis qu'un second port (606) du tampon d'entrée différentiel (602) couplé à une seconde électrode d'entrée (104B) des au moins deux électrodes d'entrée fournit une entrée pour l'autre des premier et deuxième amplificateurs à haute impédance ;
le circuit soustracteur (608) comprend un troisième amplificateur configuré pour soustraire les sorties des deux amplificateurs à haute impédance pour éliminer un quelconque signal commun à la fois au signal d'entrée au premier port (604) et au signal d'entrée au second port (606) ;
la différence de signal entre le signal d'entrée au premier port (604) et le signal d'entrée au second port (606) forme un signal de ligne de base (614), qui est envoyé à la fois à l'amplificateur (610) et au circuit de restauration de ligne de base (612) ;
le circuit de restauration de ligne de base (612) est configuré pour comparer le signal de ligne de base (614) et un signal de tension de polarisation (628) provenant du circuit de polarisation de tension (616) et pour renvoyer un signal de correction (626) obtenu au circuit soustracteur (608) ; et
le signal de tension de polarisation (628) est utilisé pour corriger des variations de la ligne de base dans le signal de ligne de base (614).

2. Système de surveillance électrocardiographique (100, 101) selon la revendication 1, dans lequel la pluralité d'électrodes (104, 104A, 104B, 104C, 154, 154A, 154B et 154C) comprend au moins trois électrodes d'entrée (154A, 154B et 154C) configurées pour envoyer des signaux au processeur de signaux (106, 156).

3. Système de surveillance électrocardiographique (100, 101) selon la revendication 1 ou 2, dans lequel la pluralité d'électrodes (104, 104A, 104B, 104C, 154, 154A, 154B et 154C) comprend une électrode (154C) configurée comme une borne de terre.

4. Système de surveillance électrocardiographique (100, 101) selon l'une quelconque des revendications précédentes,
dans lequel le processeur de signaux (106, 156) comprend en outre un filtre passe-bas (1561pf, 620) couplé à l'amplificateur (156a, 610) et au circuit de polarisation de tension (156vbc, 616), et/ou un circuit neutre (156nc) couplé au circuit de polarisation de tension (156vbc, 616), le circuit neutre (156nc, 618) couplé à une électrode de borne de terre (154C) de la pluralité d'électrodes (104, 104A, 104B, 104C, 154, 154A, 154B et 154C).

5. Système de surveillance électrocardiographique (100, 101) selon la revendication 4,
dans lequel le processeur de signaux (106, 156) comprend en outre un convertisseur analogique-numérique (156adc, 622) couplé au filtre passe-bas (1561pf, 620) et à l'émetteur (108, 158).

6. Système de surveillance électrocardiographique (100, 101) selon l'une quelconque des revendications précédentes, dans lequel la distance entre chaque centre de la pluralité d'électrodes (104, 104A, 104B, 104C, 154, 154A, 154B et 154C) est inférieure à 5 cm.

7. Système de surveillance électrocardiographique (100, 101) selon l'une quelconque des revendications précédentes, dans lequel la forme du boîtier (102, 152) présente une plus grande longueur (202) inférieure à 7 cm.

8. Système de surveillance électrocardiographique (100, 101) selon l'une quelconque des revendications précédentes, dans lequel une distance égale (304, 306 et 308) existe entre chaque centre de la pluralité d'électrodes (104, 104A, 104B, 104C, 154, 154A, 154B et 154C).

9. Système de surveillance électrocardiographique (100, 101) selon l'une quelconque des revendications précédentes, dans lequel la pluralité d'électrodes (104, 104A, 104B, 104C, 154, 154A, 154B et 154C) sont agencées de sorte que chaque électrode (104, 104A, 104B, 104C, 154, 154A, 154B et 154C) est située au niveau d'un sommet d'un triangle,
dans lequel le triangle est de préférence équilatéral ou isocèle.

10. Système de surveillance électrocardiographique (100, 101) selon l'une quelconque des revendications précédentes, comprenant en outre :
un récepteur (112, 162) pour recevoir des signaux de l'émetteur (108, 158) ;
un processeur (116, 166) couplé au récepteur (112, 162), le processeur (116, 166) étant configuré pour traiter les signaux reçus de l'émetteur (108, 158) ; et
un afficheur (118, 168) couplé au processeur (116, 166), l'afficheur (118, 168) étant configuré pour montrer des données d'ECG provenant des signaux reçus par le récepteur (112, 162).

11. Système de surveillance électrocardiographique (100, 101) selon la revendication 10, dans lequel le récepteur (112, 162), le processeur (116, 166) et l'afficheur (118, 168) sont situés dans une plateforme (120, 170) éloignée du boîtier (102, 152).

12. Système de surveillance électrocardiographique (100, 101) selon la revendication 11, dans lequel la plateforme (120, 170) comprend un ou plusieurs quelconques des dispositifs suivants : un serveur informatique (1406), un dispositif mobile (1404) et un terminal informatique (1408).

13. Système de surveillance électrocardiographique (100, 101) selon la revendication 12, dans lequel le dispositif mobile (1404) comprend un ou plusieurs quelconques des dispositifs suivants : un téléphone portable, un assistant personnel, une tablette et un ordinateur portable.

14. Système de surveillance électrocardiographique (100, 101) selon l'une quelconque des revendications précédentes, comprenant en outre une source d'alimentation (115, 165) pour alimenter le processeur de signaux (106, 156) et l'émetteur (108, 158).

15. Système de surveillance électrocardiographique (100, 101) selon l'une quelconque des revendications précédentes, dans lequel l'émetteur (108, 158) transmet des signaux en utilisant un ou plusieurs supports quelconques des supports suivants : EDGE, bluetooth, 3G, HSDPA, LTE, WiFi, WiMax, RF faible puissance fonctionnant par câble ou dans la bande ISM.
